# EUROPEAN PATENT SPECIFICATION

(11) **EP 3 290 041 B1**
(45) Date of publication and mention of the grant of the patent: **12.08.2020**
(21) Application number: 16786138.4
(22) Date of filing: 25.04.2016
(51) Int. Cl.: A23L 2/52, A61K 31/702, A61K 38/16, A61P 1/02, A61P 43/00, A23L 2/66, A61K 8/64, A61Q 5/12, A61Q 11/00, A61K 38/40, A61K 8/73, A61K 38/44, A61K 45/06, A61K 33/00, A61K 33/06, A23L 27/00, A23L 33/10, A23L 33/19

(54) **ORAL CARE COMPOSITION**
MUNDPFLEGEZUSAMMENSETZUNG
COMPOSITION DE SOIN BUCCAL

(30) Priority: 27.04.2015 JP 2015090444; 07.05.2015 JP 2015095075; 18.06.2015 JP 2015122770; 20.11.2015 JP 2015227257; 31.03.2016 JP 2016070214
(43) Date of publication of application: 07.03.2018
(73) Proprietor: Japan Mode Co. Ltd., Tokyo 120-0026 (JP)
(72) Inventor: KAWASE, Tarou, Koshigaya-shi Saitama 343-0025 (JP); KAWASE, Ryuji, Koshigaya-shi Saitama 343-0025 (JP); KAWASE, Akemi, Koshigaya-shi Saitama 343-0025 (JP)
(74) Representative: Rings, Rolf
(86) International application number: PCT/JP2016/002171
(87) International publication number: WO 2016/174861

(56) References cited:
- EP-B1- 1 363 580
- WO-A1-2010/061932
- JP-A- H0 912 473
- JP-A- 2002 322 088
- JP-A- 2002 325 557
- JP-A- 2009 102 353
- JP-A- 2014 080 381
- JP-B2- 5 461 198

## Description

### Technical Field

The present invention relates to an oral care composition, that is suitable for maintaining the health of an oral cavity by inducing secretion of large amount of saliva exhibiting a bactericidal and antibacterial activity in the oral cavity.

### Background Art

Saliva has a function of maintaining an oral cavity in wet state, facilitating digestion of food, and cleaning an oral cavity based on physical or biochemical mechanism, or the like. Furthermore, saliva plays a role of preventing dental caries (i.e., tooth decay) by maintaining pH of an oral cavity at constant level and maintaining pH of an oral cavity in more neutral state according to the action of bicarbonate ions (HCO₃⁻) in saliva. Namely, saliva plays an important role in eating and maintaining of the health of an oral cavity.

In recent years, however, population with so-called dry mouth (xerostomia) increases due to dry oral tissues caused by decreased secretion amount of saliva. In case of having an onset of dry mouth, symptoms like having a difficulty in swallowing food are accompanied, and also a decrease in antibacterial activity is caused due to decreased amount of saliva . As a result, proliferation of microbes which consume food debris as a nutrition source is caused so that an environment inside an oral cavity deteriorates, an occurrence of an oral cavity disease like tooth decay and periodontitis is yielded, and also unpleasant sticky feeling in an oral cavity and a problem in swallowing or talking are caused accordingly.

In addition, with regard to the decrease in saliva to cause an occurrence of dry mouth, various causes including aging or side effects of taking medicines are suspected as well as the influence by a disease of salivary gland or a systemic disease. Due to such reasons, it is often difficult to completely eradicate those causes while treating the dry mouth.

Under the circumstances, as a conventional method for treating the dry mouth, a method of promoting saliva secretion by applying a salivation promoting agent consisting of an acid, a plant extract, or the like or a food product containing them is suggested in addition to a method of physically stimulating saliva gland based on saliva gland massage or chewing for direct promotion of saliva secretion (see Patent Literature 1, for example). Furthermore, as a method for moisturizing an oral cavity, a method of holding in an oral cavity a composition which contains a moisturizing component having high molecular weight component for moisturization of an oral cavity is also suggested.

### Citation List

### Patent Literature

Patent Literature 1: JP 11-71253 A
Patent Literature 2: JP 2007-84501 A
Patent Literature 3: JP5461198 B2

Patent Literature 3 discloses anti-caries compositions, comprising phosphorylated oligosaccharide of calcium, most preferred at 5 weight %. As an anti-caries composition such composition is an oral care composition improving the health of the oral cavity.

### Summary of Invention

The scope of the present invention is defined by the appended claims. Any other disclosure in the present description, regardless of whether it is indicated as preferred or exemplified, does not form part of the present invention. References to "methods of treatment" present in the description do not constitute embodiments of the inventions, but mere disclosure.

### Technical Problem

However, although the dry mouth symptom can be surely improved by promoting secretion of saliva according to the aforementioned technique of prior art, there is a problem that the secretion amount of saliva cannot be dramatically increased by it. Furthermore, the aforementioned technique of prior art is not suitably constituted for a case in which the use is made in combination with an eye drop or a nasal drop or a case for having easy intake of a tablet even by a person with dry mouth symptom based on coating of the periphery of a tablet with a component for secreting saliva. Furthermore, the aforementioned technique of prior art is not suitably constituted for a case in which production and selling is made after mixing with a granular or powder pharmaceutical preparation or for a case in which use is made after mixing with cosmetics, shampoo, rinse, spray, gel, or the like. Furthermore, the aforementioned technique of prior art is not suitably constituted for a case in which combination is made with a food or a drink.

Namely, there has been a need for having a constitution that is suitable for an improvement of dry mouth symptom by dramatically increasing the secretion amount of saliva and also for an application of the same composition to various uses other than the improvement of dry mouth.

In this regard, the present invention is devised in view of the aforementioned problems, and object of the present invention is to provide an oral care composition which is suitable for further improvement of dry mouth symptom by dramatically increasing the secretion amount of saliva and for application of the almost same composition to other use.

### Solution to Problem

An oral care composition according to a first invention is an oral care composition for improving the health of an oral cavity wherein the composition is to be held in an oral cavity, the oral care composition including, in terms of % by mass relative to the total mass of the composition, phosphoryl oligosaccharides of calcium: 1% to less than 30% and lactoferrin: 0.01 to 10%.

According to an oral care composition according to a second invention, in the first invention, sodium bicarbonate: 1% to 20% is further included.

According to an oral care composition according to a third invention, in the first or second invention, potassium carbonate or potassium hydrogen carbonate: 1% to 5% is further included.

According to an oral care composition according to a fourth invention, in the first invention, magnesium carbonate: 1% to 5% is further included.

According to an oral care composition according to a fifth invention, in the first invention, sucralose: 1% to 5% is further included.

### Advantageous Effects of Invention

According to the present invention having the constitution described above, by having the oral care composition held in an oral cavity of a subject, phosphoryl oligosaccharides of calcium and lactoferrin contained in the oral care composition work in synergistic manner due to the presence of each other and saliva is secreted in a large amount. At that time, by containing the aforementioned sodium bicarbonate, potassium carbonate, or potassium hydrogen carbonate, secretion of saliva is further promoted.

Accordingly, as secretion of saliva in an oral cavity is dramatically promoted, digestion of food is promoted and, as food debris in an oral cavity is cleaned, the self-cleaning activity increases. Furthermore, a bactericidal activity is exhibited in an oral cavity and also the pH in an oral cavity is maintained in a more alkaline region, and thus an occurrence or a progress of dental caries can be prevented and also the remineralization activity can be exhibited. Furthermore, as secretion of saliva in an oral cavity is promoted by the oral care composition to which the present invention is applied, it is also possible to alleviate a dry mouth symptom which is frequently shown in elderly people or the like.

Furthermore, the present invention can have a constitution that is suitable for a case in which the use is made in combination with an eye drop or a nasal drop or for a case for having easy intake of a tablet even by a person with dry mouth symptom based on coating of the periphery of a tablet with a component for secreting saliva. Furthermore, the present invention can have a constitution that is suitable for a case in which production and selling is made after mixing with a granular or powder pharmaceutical preparation or for a case in which use is made after mixing with cosmetics, shampoo, rinse, spray, gel, or the like. Furthermore, the present invention can have a constitution that is suitable for a case in which combination is made with a food or a drink.

Namely, according to the present invention with the constitution described above, it is possible to have a constitution that is suitable for an improvement of dry mouth symptom by dramatically increasing the secretion amount of saliva and also for an application of the same composition to various uses other than the improvement of dry mouth.

In particular, because secretion of a large amount of saliva can be achieved by the present invention, according to holding in mouth even in an area with no available water like disaster area, an occurrence or a progress of dental caries can be prevented without tooth brushing. Furthermore, by holding the oral care composition to which the present invention is applied in mouth of a person with difficulty in tooth brushing like the elderly or disabled, an occurrence or a progress of dental caries can be prevented without tooth brushing.

### Brief Description of Drawings

Fig. 1 is a drawing for explaining an example in which the present invention is applied to a tablet.
Fig. 2 is a drawing for explaining the overall flow of the method for producing a pharmaceutical product to which the present invention is applied.
Fig. 3 is a drawing for explaining the overall flow of the method for producing a pharmaceutical product to which the present invention is applied, in which designing step is introduced.
Fig. 4 is a drawing for explaining the overall flow of the method for producing a chemical composition to which the present invention is applied.
Fig. 5 is a drawing for explaining the overall flow of the method for producing a chemical composition to which the present invention is applied, in which designing step is introduced.
Fig. 6 is a drawing illustrating the oral fitting body which is used for an actual application.

### Description of Embodiments

To solve the problems described above, by focusing on the activities exhibited by saliva, inventors of the present invention invented an oral care composition which enables secretion of saliva in large quantities and maintaining of the health of an oral cavity. The main activities exhibited by saliva are as described below.

In saliva, an amylase is included as a digestive enzyme. By degrading saccharides included in foods, the amylase exhibits the effect of facilitating the absorption in living body. By including the amylase as an enzyme, saliva exhibits the activity of helping the digestion of foods.

Furthermore, according to mixing between foods present in an oral cavity with saliva, a food mass with suitable size can be yielded. Due to such reasons, saliva can exhibit the activity of helping food swallowing. Furthermore, saliva exhibits a so-called self-cleaning activity of cleaning food debris remained in an oral cavity. Namely, with good chewing, the amount of saliva for secretion is increased and also higher self-cleaning activity is obtained.

Also included in saliva is a lysozyme which exhibits an antibacterial activity against microbes which enter via a mouth or a nose from the outside. Saliva also plays a role of cleaning the inside of an oral cavity, and according to secretion of saliva in large quantities, contaminations present inside an oral cavity can be removed, and it also becomes possible to prevent bad breath.

Saliva also exhibits the role of maintaining pH inside an oral cavity at constant level, i.e., so-called buffering activity. According to the action of bicarbonate ions (HCO₃⁻) in saliva, pH inside the oral cavity is maintained such that it is closer to a neutral value. In general, dental caries (tooth decay) starts from so-called demineralization at which calcium or minerals on a surface of a tooth are dissolved as pH inside the oral cavity shifts to an acidic region according to production of a large amount of acid in plaque. However, as saliva is secreted in large quantities, inside of the oral cavity can be rapidly brought back to a neutral region based on the buffering activity of saliva itself.

Saliva furthermore exhibits a so-called remineralization activity by which calcium or minerals on a surface of a tooth which have been dissolved by an acid are replenished and recovered.

Inventors of the present invention conducted intensive studies on conditions for having more suitable exhibition of the activities of saliva. As a result, it was experimentally shown that, by preparing an oral care composition constituted with the following components and holding it in an oral cavity of a subject, secretion of saliva in large quantities can be achieved. As a result, it was newly found that, by mixing phosphoryl oligosaccharides of calcium with lactoferrin, the secretion amount of saliva can be dramatically increased based on a synergistic effect between them. Phosphoryl oligosaccharides of calcium is a material which is hardly dissolved in water while lactoferrin is a material which is dissolved easily in water. Namely, the present invention is based on an idea which did not exist before, i.e., phosphoryl oligosaccharides of calcium and lactoferrin which are different from each other in terms of hydration property are admixed with each other to give a single oral care composition.

Namely, the oral care composition according to the present invention is an oral care composition for improving the health of an oral cavity when it is held in the oral cavity, in which the oral care composition contains, in terms of % by mass relative to the total mass of the composition, 1 to 30% of phosphoryl oligosaccharides of calcium and 0.01 to 10% lactoferrin. The oral care composition may also contain 1 to 5% of magnesium carbonate. The oral care composition may also contain 1 to 5% of sucralose. Furthermore, the oral care composition to which the present invention is applied may contain 1 to 20% of sodium bicarbonate or 1 to 5% of potassium carbonate or potassium hydrogen carbonate.

Furthermore, the oral care composition to which the present invention is applied may also contain, as a remainder, at least any one of digestion resistant dextrin, champignon extract powder, xylitol, fragrance, fine granular silicon dioxide, and calcium stearate.

Hereinbelow, the reasons for limiting the components and content of the components of the oral care composition to which the present invention is applied are explained. Furthermore, content of each component is expressed in terms of % by mass relative to the total mass of the composition, and, for expressing the % by mass, it is simply described as %.

### Phosphoryl oligosaccharides of calcium: 1 to 30%

Phosphoryl oligosaccharides of calcium is a component derived from starch having potatoes from Hokkaido as a raw material. Potato starch has a part bound with phosphate group, and, as the starch is reacted with an enzyme followed by extraction and purification and prepared as a calcium salt, phosphoryl oligosaccharides of calcium is yielded. By holding this phosphoryl oligosaccharides of calcium in an oral cavity, the activity of secreting large quantities of saliva can be obtained. Furthermore, other than that this phosphoryl oligosaccharides of calcium exhibits the role of calcium source, it is not a nutrition supply source for Streptococcus mutans as a dental caries-causing microbe and it can exhibit the role of suppressing pH decrease in an oral cavity based on its buffering activity. Furthermore, as the dissolved calcium ions are increased in saliva, phosphoryl oligosaccharides of calcium can also promote remineralization of teeth.

If the content of phosphoryl oligosaccharides of calcium is less than 1%, the activity intrinsic to phosphoryl oligosaccharides of calcium is not exhibited so that the aforementioned desired effect cannot be exhibited. On the other hand, if the content of phosphoryl oligosaccharides of calcium is more than 30%, the effect is saturated and at the same time higher raw material cost will be necessary as phosphoryl oligosaccharides of calcium is contained in a large amount. Furthermore, if phosphoryl oligosaccharides of calcium is added in a large amount, i.e., more than 30%, strong toxicity to an infant will be caused. As such, the content of phosphoryl oligosaccharides of calcium is set at 1 to 30%.

### Lactoferrin: 0.01 to 10%

Lactoferrin is an iron-binding glycoprotein with molecular weight of about 80,000 which is widely present in the body of an animal. As for the lactoferrin, a product obtained by using, as a milk source, those isolated from colostrum, transitional milk, normal milk, or late lactation milk of a mammal (for example, human, cow, goat, sheep, and horse) or non-fat milk, milk whey, or the like as a processed product of such milk, followed by isolation from those sources by using a known isolation and purification method like ion exchange chromatography can be used. Furthermore, lactoferrin can be either a product produced from a plant (e.g., tomato, rice, and tobacco) or a product obtainable by genetic engineering. Furthermore, as for the lactoferrin, a commercially available product can be used or it can be used after producing it by a known method. Lactoferrin can be used either singly or in suitable combination of two or more kinds thereof. By holding this lactoferrin in an oral cavity, the activity of secreting large quantities of saliva is caused.

The main function exhibited by lactoferrin includes introduction of immunologic tolerance, enhanced recognition of foreign materials, inhibition of angiogenesis, and enhanced opioid activity. According to introduction of the immunologic tolerance, it is expected to have an effect of improving an autoimmune disease and various allergies. According to the enhanced recognition of foreign materials, it is expected to have an effect for improving an infectious disease, in particular, an opportunistic infectious disease. Furthermore, by also exhibiting the effect of inhibiting angiogenesis which is induced by inflammation, lactoferrin is effective for an angiogenic disease including cancer. Furthermore, as the effect of an endogenous opioid referred to as intracerebral narcotics is enhanced by it, lactoferrin can exhibit an analgesic effect against pains.

In addition to the aforementioned saliva secretion activity, lactoferrin can also exhibit the effect on repairing of conjunctival mucous membrane and rejuvenation of lachrymal gland. Due to such reasons, lactoferrin is also expected to have an effect of ameliorating so-called dry eye according to addition of an eye drop of lactoferrin. There is also a possibility that lactoferrin can treat or prevent a neurodegenerative disease like Alzheimer's disease.

If the content of lactoferrin is less than 0.01%, the activities intrinsically belonging to lactoferrin not exhibited so that the aforementioned certain effect cannot be obtained. On the other hand, if the content of lactoferrin is more than 10%, the effect is saturated and at the same time higher raw material cost will be necessary as it is contained in a large amount. Furthermore, if the content of lactoferrin is more than 10%, a high blood sugar level will be resulted in a patient who has taken the lactoferrin. Due to such reasons, the content of lactoferrin is set at 0.01 to 10%.

According to the present invention, it is also possible that the following components are further contained after the aforementioned phosphoryl oligosaccharides of calcium and lactoferrin are being contained.

### Sodium bicarbonate: 1 to 20%

Sodium bicarbonate (i.e., sodium hydrogen carbonate) is generally referred to as baking soda, and it is widely used as baking powder and an additive for soft drink or the like in the field of various food products and it is also used as a dialysis agent, a gastrointestinal pharmaceutical, or the like in the field of pharmaceuticals, as well as, as an extinguishing agent, a bath agent, a cleaning agent or the like. By holding sodium bicarbonate in an oral cavity, the activity of secreting large quantities of saliva is caused.

If the content of sodium bicarbonate is less than 1%, the activities intrinsically belonging to sodium bicarbonate are not exhibited so that the aforementioned certain effect cannot be obtained. On the other hand, if the content of sodium bicarbonate is more than 20%, the effect is saturated. Due to such reasons, the content of sodium bicarbonate is set at 1 to 20%.

In addition, the content of sodium bicarbonate is preferably set at 6 to 16% in order to exhibit the desired effect. It is more preferably set at 10 to 16%. With regard to the example of preferred further sodium bicarbonate, the reason of having the upper limit of 16% is that, if sodium bicarbonate is added at more than 16%, a composition easily causing vomiting with a feeling of nausea can be yielded.

### Potassium carbonate: 1 to 5%

In many fields including pharmaceuticals, food products, cosmetics, or the like, potassium carbonate has long been used as a versatile component, and it is blended, as an antiacid or an inhibitor for acidosis, in pharmaceuticals, or used as a component of an oral rehydration agent. Furthermore, potassium carbonate can also make saliva alkaline or exhibit the activity of a buffering agent for maintaining saliva in an alkaline state . By holding potassium carbonate in an oral cavity, the activity of secreting large quantities of saliva is caused.

If the content of potassium carbonate is less than 1%, the activities intrinsically belonging to potassium carbonate cannot be exhibited so that the aforementioned certain effect cannot be obtained. On the other hand, if the content of potassium carbonate is more than 5%, the effect is saturated. Due to such reasons, the content of potassium carbonate is set at 1 to 5%. Furthermore, as a substitute of potassium carbonate, it is also possible to add potassium hydrogen carbonate.

Furthermore, according to the present invention, the following components may be contained, if necessary.

### Phosphoric acid source compound: 1 to 3%

Phosphoric acid source compound is a compound which is selected from a group consisting of phosphoric acid, sodium phosphate, potassium phosphate, polyphosphoric acid, and cyclic phosphate. Among them, polyphosphoric acid is a compound which is formed by condensation of two or more phosphoric acids, and it is often used as a vehicle, a discoloration inhibitor, or the like in a food product. Polymerization degree of polyphosphoric acid can have any value as long as it is 2 or higher, and it is 2 or higher and 10 or lower, for example. Examples of the polyphosphoric acid include pyrophosphoric acid, triphosphoric acid, trimetaphosphoric acid, tetrametaphosphoric acid, and cyclic polyphosphoric acid. Examples of the cyclic phosphoric acid include hexametaphosphoric acid. Polyphosphoric acid can suppress a decrease in gut barrier function and also can exhibit the activity of restoring the gut barrier function. Polyphosphoric acid is synthesized from ATP by polyphosphoric acid kinase in microorganisms including E. coli. By holding polyphosphoric acid in an oral cavity, the activity of secreting large quantities of saliva is caused. This phosphoric acid source compound is preferably contained within a range of 1 to 3%.

### Magnesium carbonate: 1 to 5%

Magnesium carbonate is mainly used as an abrasive of tooth paste or as an antiacid. By mixing magnesium carbonate, the activity of secreting large quantities of saliva is caused. If the content of magnesium carbonate is less than 1%, related saliva secretion effect cannot be exhibited. On the other hand, if the content of magnesium carbonate is more than 5%, the effect is saturated. Due to such reasons, the content of magnesium carbonate is set at 1 to 5%.

### Sucralose: 1 to 5%

Sucralose is one of artificial sweetening agents generated from sugar. Sucralose has a sweetening power that is about 600 times that of sugar, and it is easily dissolved in water and excellent in terms of stability. Furthermore, unlike sugar, sucralose is not digested and absorbed as carbohydrate in living body while it exhibits mild taste similar to sugar. As such, it has absolutely no physiological calorie. Due to such reasons, sucralose is applied to various drink compositions and food compositions including soft drink and ice cream. Furthermore, according to the mild taste exhibited by sucralose, the activity of secreting large quantities of saliva is exhibited. Furthermore, if the content of sucralose is less than 1%, related saliva secretion effect cannot be exhibited. On the other hand, if the content of sucralose is more than 5%, the effect is saturated and at the same time higher raw material cost will be necessary as it is contained in a large amount. Due to such reasons, the content of sucralose is set at 1 to 5%.

Furthermore, according to the present invention, the following components may be contained, if necessary.

### Components of remainder

Digestion resistant dextrin indicates water soluble dietary fiber having a property of digestion resistance, which results from a treatment of indigestible dextrin that is obtained by subjecting starch derived from plant like corn, wheat, rice, beans, yams, and tapioca to acid addition and/or heating, and, if necessary, a treatment of the indigestible dextrin with α amylase and/or glucoamylase, followed by desalting and decoloration, if necessary. In the oral care composition to which the present invention is applied, it is used as a bulking agent in a case in which the composition is prepared as a tablet. Content of the digestion resistant dextrin is not particularly limited, and it can be added in an appropriate amount.

Champignon extract powder is a component extracted from mushroom, and it is added in an appropriate amount to remove smells like bad breath.

Xylitol is pentose sugar alcohol present in nature, and with regard to those added in the present invention, any known one can be used as a supply source thereof. According to addition of xylitol, easy intake can be achieved for a case in which the oral care composition is prepared as a tablet. Content of the xylitol is not particularly limited, and it can be added in an appropriate amount.

A fragrance is obtained by combining a plurality of natural fragrances which have been extracted from various plants or some animals, or synthetic fragrances which have been synthesized chemically. For example, easy intake can be achieved if an orange fragrance is added to prepare the oral care composition as a tablet. Furthermore, by adding a fragrance of menthol, exhibition of refreshing feel can be achieved at the time of intake. Content of the fragrance is not particularly limited, and it can be added in an appropriate amount.

Fine granular silicon dioxide indicates sodium silicic acid in fine granular form that is obtained by degradation with hydrochloric acid or sulfuric acid. Although it is added in an appropriate amount to adjust hardness, addition of the fine granular silicon dioxide is not necessarily essential.

Calcium stearate indicates a calcium salt of stearic acid and palmitic acid in which palm-derived fatty acid having effects of lubrication, fluidization, or prevention of solidification of a product in powder phase or having effects of emulsifying the product texture and increasing the viscosity is used. Content of the calcium stearate is not particularly limited, and it can be added in an appropriate amount.

The oral care composition to which the present invention is applied may be achieved either by preparing each component with the aforementioned content in a solid phase like tablet or powder or by preparing it as a liquid.

As the oral care composition to which the present invention is applied is held in the oral cavity of a subject, phosphoryl oligosaccharides of calcium and lactoferrin contained in the oral care composition work in synergistic manner due to the presence of each other and saliva is secreted in a greater amount. At that time, by having the aforementioned sodium bicarbonate, potassium carbonate, or potassium hydrogen carbonate contained in the composition, secretion of saliva is further promoted. Because the calcium part of phosphoryl oligosaccharides of calcium rapidly binds to lactoferrin to yield lactoferrin with a structure that is hardly breakable by a proteinase, it becomes possible to stabilize it in terms of the structure.

Accordingly, secretion of saliva in an oral cavity is promoted so that digestion of food can be facilitated and a higher activity of self-cleaning un-eaten debris remaining in an oral cavity can be obtained. Furthermore, as the bactericidal activity is exhibited in an oral cavity and pH in an oral cavity is maintained at more neutral value, an occurrence or a progress of dental caries can be prevented and also the remineralization activity can be exhibited.

According to the present invention, because secretion of saliva in large quantities can be achieved, in particular, even in an area with no available water like disaster area, an occurrence or a progress of dental caries can be prevented without tooth brushing according to holding it in a mouth. Furthermore, by having the oral care composition to which the present invention is applied held in mouth of a person who has a difficulty in tooth brushing like the elderly or disabled, an occurrence or a progress of dental caries can be prevented without tooth brushing.

Furthermore, according to the oral care composition to which the present invention is applied, secretion of saliva in an oral cavity is promoted so that it is also possible to alleviate a dry mouth symptom which is frequently shown in elderly people or the like.

Furthermore, in case of preparation of a pharmaceutical product, any pharmaceutical agent can be used as a pharmaceutical component of the product.

The auxiliary component is contained at 0.01 to 50% in terms of % by mass relative to the total mass of a pharmaceutical product. If the auxiliary component is contained at less than 0.01% in terms of % by mass relative to the total mass of a pharmaceutical product, the aforementioned effect of the auxiliary component cannot be exhibited. On the other hand, if the auxiliary component is contained at more than 50% in terms of % by mass relative to the total mass of a pharmaceutical product, the effect is saturated.

Furthermore, it is also disclosed that the composition of the invention can be applied to an eye drop in which the components at the aforementioned blending ratio of the oral care composition are mixed. In case of preparing an eye drop, an eye drop liquid containing the auxiliary components at the aforementioned blending ratio of the oral care composition and an eye drop component to be contained in a common eye drop is prepared. Examples of the eye drop component include an anti-inflammation agent, a vitamin agent, a vasoconstrictor, an anti-histamine agent, a mydriatic agent, a miotic agent, an intraocular pressure reducing agent, a cataract treatment agent, a steroid hormone agent, an antibiotic, a local anesthetic, a hyperemic agent, an amino acid component, and an antibacterial component, but it is not limited thereto, and any other component for an eye drop can also be mixed. Furthermore, the eye drop liquid contains water and a preservative in addition to the eye drop component.

The auxiliary component is contained at 0.01 to 10% in terms of % by mass relative to the total mass of an eye drop liquid. Furthermore, it is also possible to have a constitution that phosphoryl oligosaccharides of calcium and lactoferrin as an auxiliary component are separately added to an eye. In that case, an eye drop liquid consisting of phosphoryl oligosaccharides of calcium, and if necessary, an eye drop component is added to one container, and an eye drop liquid consisting of lactoferrin, and if necessary, an eye drop component is added to the other container. The eye drop liquid in any one container is added to an eye, and after lapse of short period of time, the eye drop liquid in the other container is added to the eye. Accordingly, an overlap between the time point for adding phosphoryl oligosaccharides of calcium and the time point for adding lactoferrin is avoided so that mixing of the phosphoryl oligosaccharides of calcium with lactoferrin in an eye can be prevented.

According to the eye drop liquid obtained by the constitution described above, based on the lactoferrin's activity of repairing conjunctival mucous membrane and rejuvenating lacrimal gland cells, it becomes possible to improve so-called dry eye. Furthermore, it is also possible that only the oral care composition in liquid phase itself is prepared as an eye drop liquid.

A further disclosure is that an application can also be made to a nasal drop in which the components at the aforementioned blending ratio of the oral care composition are mixed. In case of preparing a nasal drop, a nasal drop liquid containing the auxiliary components at the aforementioned blending ratio of the oral care composition and a nasal drop component to be contained in a common nasal drop is prepared. Examples of the nasal drop component include a vasoconstrictor, a vasodilator, an anti-histamine agent, a steroid, but it is not limited thereto, and any other component for a nasal drop can also be mixed.

The auxiliary component is contained at 0.01 to 10% in terms of % by mass relative to the total mass of a nasal drop liquid. Furthermore, it is also possible to have a constitution that phosphoryl oligosaccharides of calcium and lactoferrin as an auxiliary component are separately added as a nasal drop. In that case, a nasal drop liquid consisting of phosphoryl oligosaccharides of calcium, and if necessary, a nasal drop component is added to one container, and a nasal drop liquid consisting of lactoferrin, and if necessary, a nasal drop component is added to the other container. The nasal drop liquid in any one container is added as a nasal drop, and after lapse of short period of time, the nasal drop liquid in the other container is added as a nasal drop. Accordingly, an overlap between the time point for adding phosphoryl oligosaccharides of calcium and the time point for adding lactoferrin is avoided so that mixing of the phosphoryl oligosaccharides of calcium with lactoferrin in a nose can be prevented.

According to the nasal drop liquid having the constitution described above, once lactoferrin is added as a nasal drop, it passes a blood brain barrier through capillaries of a nose, and according to an exhibition of the activity of lactoferrin-related calcium ions to propagate in brain blood, a deficiency of calcium ions in brain blood that is specific to Alzheimer is resolved so that it becomes possible to ameliorate Alzheimer's disease. Furthermore, it is also possible that the oral care composition in liquid phase itself is prepared as a nasal drop liquid.

A further disclosure is that an application can also be made to a cosmetic product in which the components at the aforementioned blending ratio of the oral care composition are mixed. In case of preparing a cosmetic product, a cosmetic product containing the auxiliary components at the aforementioned blending ratio of the oral care composition and a cosmetic component to be contained in common cosmetics is prepared. Examples of the cosmetic product include a foundation or a creme, a cosmetic liquid other than those for application to a skin, and examples of the component for a cosmetic product include a so-called whitening component such as albutin, fullerene, tranexamic acid, hydroquinone, coix seed extract, or placenta extract, a so-called vitamin C derivative such as Na ascorbyl phosphate, Mg ascorbyl phosphate, ascorbyl tetrahexyldecanoic acid, or ascorbyl palmitate, a moisturizing component such as Na hyaluronic acid, water soluble collagen, placenta extract, ceramide 2, ceramide 3, or ceramide 6, and an anti-aging component such as astaxanthin, coenzyme Q10, EGF (human oligopeptide), platinum nano colloid, tocopheryl retinoic acid, or retinol, but they are not limited thereto, and any other component for a cosmetic product can also be mixed.

The auxiliary component is contained at 0.01 to 20% in terms of % by mass relative to the total mass of a cosmetic product. Furthermore, it is also possible to have a constitution that phosphoryl oligosaccharides of calcium and lactoferrin as an auxiliary component are separately applied to skin. In that case, a cosmetic product consisting of phosphoryl oligosaccharides of calcium, and if necessary, a cosmetic component is added to one container, and a cosmetic product consisting of lactoferrin, and if necessary, a cosmetic component is added to the other container. The cosmetic product in any one container is applied to skin, and after lapse of short period of time, the cosmetic product in the other container is applied to the skin. Accordingly, an overlap between the time point for applying phosphoryl oligosaccharides of calcium and the time point for applying lactoferrin is avoided so that mixing of the phosphoryl oligosaccharides of calcium with lactoferrin on a skin surface can be prevented.

According to the cosmetic product having the constitution described above, as calcium ions of phosphoryl oligosaccharides of calcium are associated with lactoferrin, the activity of infiltrating each layer of skin is exhibited, and also, as the activity of functioning as calcium ions that are locally present in a granular layer of epidermis is exhibited, it becomes possible to improve the calcium localization in epidermis. As a result, symptoms like rough skin, darkish feeling, and dry skin can be improved. Furthermore, according to the present invention, it is also possible that only the oral care composition in creme state or powder state itself can be prepared as a cosmetic product.

A further disclosure is that an application can also be made to a composition for cleansing skin or hair like hair shampoo, body shampoo, cleansing agent, and facial foam in which the components at the aforementioned blending ratio of the oral care composition are mixed. In case of preparing a composition for cleansing skin or hair, a composition for cleansing skin or hair containing the auxiliary components at the aforementioned blending ratio of the oral care composition and a cleansing component to be contained in common composition for cleansing skin or hair is prepared. Furthermore, this composition for cleansing skin or hair is prepared as powder, solid, paste, or liquid, for example. In the following examples, explanations are given for an exemplary case in which the composition is prepared as a liquid. The cleansing component is mainly composed of water and a surface active agent. Examples of the surface active agent include a higher alcohol-based surface active agent, an amino acid-based surface active agent, a betain-based surface active agent (i.e., amphoteric surface active agent), a non-ionic surface active agent (i.e., non-ionic type surface active agent), and a natural surface active agent. To the cleansing component, silicone, menthol, a moisturizing component, a soap, camellia oil, or the like are also added.

The auxiliary component is contained at 0.1 to 10% in terms of % by mass relative to the total mass of a composition for cleansing skin or hair. According to cleansing of hair or skin with this composition for cleansing skin or hair, an activity of delivering calcium ions and lactoferrin to an inside of hair or an epidermis layer of skin is exhibited so that it becomes possible to improve the quality of hair or skin.

A further disclosure is that an application can also be made to a hair treatment product like rinse, conditioner, treatment, and hair styling product (including gel, mousse, and spray) in which the components at the aforementioned blending ratio of the oral care composition are mixed. In case of preparing a hair treatment product, a hair treatment product containing the auxiliary components at the aforementioned blending ratio of the oral care composition and a hair treatment component is prepared. The hair treatment component is constituted in the form of powder, solid, paste, or liquid, for example. In the following examples, explanations are given for an exemplary case in which the composition is prepared as a liquid. In case of an application to rinse, conditioner, or treatment, the hair treatment component is purified water, reduced water, glycerin, squalene, a moisturizing agent, an oily component, aminoethyl aminopropylmethicone dimethicone, higher alcohol, plant seed oil, meadowfoam estolide, cholesterol, aloe extract, olive oil fatty acid glycerin, biohyaluronic acid, or the like. Furthermore, in case of an application to a hair styling product, the hair treatment component is composed of liquid oil and fat, a polymer, a solid oil and fat, a surface active agent, alcohol, water, a propellant (gas), a fragrance, a preservative, a stabilizer, silicone, or the like.

The auxiliary component is contained at 0.1 to 10% in terms of % by mass relative to the total mass of a hair treatment product. According to treatment of hair with the hair treatment product, an activity of fixing lactoferrin and phosphoryl oligosaccharides of calcium ions to hair roots based on electrolysis of an ion channel is caused so that it becomes possible to carry out an effective hair treatment.

Furthermore, according to an example, an application can also be made to a food composition in which the components at the aforementioned blending ratio of the oral care composition are mixed. In case of preparing a food composition, a food composition containing the auxiliary components at the aforementioned blending ratio of the oral care composition and a food component for constituting common food products or snack is prepared. The food composition is constituted with nutrients like protein, lipid, carbohydrate, calcium, vitamin, and carotene. The snack described herein includes a luxury grocery item like gum and candy.

The auxiliary component is contained at 0.01 to 30% in terms of % by mass relative to the total mass of a food composition. With this food composition, it becomes possible to have exhibition of the lactoferrin's activity of transferring to cerebrospinal fluid over the blood brain barrier in addition to general intake of nutrients, and it also becomes possible to treat and prevent a neurodegenerative disorder including Alzheimer's disease. Furthermore, as phosphoryl oligosaccharides of calcium and lactoferrin work in synergistic manner due to the presence of each other, saliva is secreted in a greater amount. As a result, dramatically increased secretion of saliva in an oral cavity is caused so that a food product that is easily edible with saliva even by a person with dry mouth symptom can be provided. Furthermore, even the un-eaten debris which remains in an oral cavity after eating can be processed by saliva with increased secretion amount, and thus it can contribute to enhance the health of an oral cavity.

Furthermore, according to an example, an application can also be made to a drink composition in which the components at the aforementioned blending ratio of the oral care composition are mixed. In case of preparing a drink composition, a drink composition containing the auxiliary components at the aforementioned blending ratio of the oral care composition and a component for drink for constituting common drinks is prepared. The drink composition includes a refresh drink, a carbonate drink, an alcohol drink and the like, and it is constituted with water, coffee, a fragrance, a coloring agent, sugar, vitamin C, salt, fruit extract, alcohols, or the like.

The auxiliary component is contained at 0.01 to 20% in terms of % by mass relative to the total mass of a drink composition. With this drink composition, it becomes possible to have exhibition of the lactoferrin's activity of transferring to cerebrospinal fluid over the blood brain barrier in addition to general intake of water and nutrients, and it also becomes possible to treat and prevent a neurodegenerative disorder including Alzheimer's disease. Furthermore, as phosphoryl oligosaccharides of calcium and lactoferrin work in synergistic manner due to the presence of each other, saliva is secreted in a greater amount. As a result, dramatically increased secretion of saliva in an oral cavity is caused, and thus it can contribute to enhance the health of an oral cavity.

Furthermore, according to an example, an application can also be made to a seasoning composition in which the components at the aforementioned blending ratio of the oral care composition are mixed. In case of preparing a seasoning composition, a seasoning composition containing the auxiliary components at the aforementioned blending ratio of the oral care composition and a component for seasoning for constituting common seasoning is prepared. This seasoning composition is prepared in the form of liquid, solid, powder, or the like, and it is represented by, other than table salt, soy sauce, sugar, vinegar, and miso, chili oil, dressing, mayonnaise, ketchup, Tabasco, pepper, seven spice blend, sweet sake, cooking wine, Japanese style seasoning (tsuyu, ponzu), or the like. However, it may be also those containing an amino acid or the like as a main component as it is represented by synthetic seasoning or the like.

The auxiliary component is contained at 0.01 to 10% in terms of % by mass relative to the total mass of a seasoning composition. With this seasoning composition, it becomes possible to have exhibition of the lactoferrin's activity of transferring to cerebrospinal fluid over the blood brain barrier in addition to general intake of water and nutrients, and it also becomes possible to treat and prevent a neurodegenerative disorder including Alzheimer's disease. Furthermore, as phosphoryl oligosaccharides of calcium and lactoferrin work in synergistic manner due to the presence of each other, saliva is secreted in a greater amount. As a result, dramatically increased secretion of saliva in an oral cavity is caused, and thus it can contribute to enhance the health of an oral cavity.

Furthermore, according to an example, it is also possible to contain lactoperoxidase: 1 to 20% in terms of % by mass relative to the total mass of auxiliary components. Like lactoferrin, lactoperoxidase is a biological defense component contained in saliva or milk, and it is an enzyme exhibiting an antibacterial activity against various microbes such as periodontal bacteria. If lactoperoxidase is less than 1% in terms of % by mass relative to the total mass of auxiliary components, more effective exhibition of the antibacterial activity cannot be obtained. On the other hand, if lactoperoxidase is more than 20% in terms of % by mass relative to the total mass of auxiliary components, the effect is saturated. Due to such reasons, to have the antibacterial activity exhibited by lactoperoxidase at suitable level, lactoperoxidase is preferably contained at 1 to 20% in terms of % by mass relative to the total mass of auxiliary components.

Furthermore, although any disclosure explained above has a 2-component system which consists of a main component (i.e., pharmaceutical component, food component, and drink component) and an auxiliary component, there is also further disclosure presented. Namely, it is also possible to have an auxiliary component only. In that case, any pharmaceutical liquid, cosmetics, food composition, drink composition, or the like consist of 100% auxiliary component in which phosphoryl oligosaccharides of calcium and lactoferrin constituting the auxiliary component are mixed at the aforementioned ratio. Furthermore, even for a case of 2-component system consisting of a main component and an auxiliary component, the content ratio of an auxiliary component is not limited to the range described above, and as long as it is between 0.1 to 100%, it can have any content ratio.

The present invention can also be applied as an agent for improving low body temperature. The agent for improving low body temperature is an agent for improving low body temperature that can enhance the body temperature increase upon administration. The agent for improving low body temperature contains an auxiliary component, and it is characterized by containing phosphoryl oligosaccharides of calcium: 1 to 30%, lactoferrin: 0.01 to 10%, and magnesium sulfate: 1 to 70% in terms of % by mass relative to the total mass of auxiliary components. In that case, it is also possible that the auxiliary component is contained at 1 to 70% in terms of % by mass relative to the total mass of the agent for improving low body temperature .

Next, with regard to the agent for improving low body temperature to which the present invention is applied, the reasons for limiting the components and content are explained. Furthermore, content of each component is expressed in terms of % by mass relative to the total mass of the auxiliary component, and, for expressing the % by mass, it is simply described as %. Furthermore, because the reason for having limitation of phosphoryl oligosaccharides of calcium: 1 to 30% and lactoferrin : 0.01 to 10% is the same as described above, only the reason for having limitation of magnesium sulfate: 1 to 70%, total of B group vitamins: 1 to 10%, and blood circulation accelerator: 10 to 30% is explained. Furthermore, the remainder related to the agent for improving low body temperature is the same as described above.

### Magnesium sulfate: 1 to 70%

Magnesium sulfate is easily dissolved in water, and upon reaction with water in esophagus, it exhibits the activity of generating heat of about 45°C. As body temperature is increased accordingly, it becomes possible to enhance the basic metabolism. As a result, an interaction with the aforementioned lactoferrin is caused so that the activity of promoting synergistically the body temperature increase is exhibited.

In particular, when dissolved in water, magnesium sulfate gives magnesium ions that are hardly ingested, but it can effectively absorb magnesium as a mineral required in human body. As a result, balance between calcium and magnesium can be optimized and so-called calcium paradox by which calcium is fixed in cells like arterial cells in which calcium is not supposed to be deposited is inhibited. In addition, magnesium sulfate has an effect of preventing arteriolosclerosis, hypertension, or the like.

Furthermore, the magnesium ions obtained by decomposition of magnesium sulfate are also a mineral that is required for the below-mentioned B group vitamins to exhibit the body temperature increasing effect in a human body.

Furthermore, the magnesium ions obtained by decomposition of magnesium sulfate also have, like calcium ions, the activity of agglomerating lactoferrin to prevent the decomposition caused by gastric juice,

When magnesium sulfate is less than 1%, it is difficult to exhibit the aforementioned effect of increasing the basal body temperature or the like. On the other hand, when magnesium sulfate is more than 70%, the effect is saturated and, at the same time, the problem of having watery stool occurs. Due to such reasons, magnesium sulfate is present at 1 to 70%.

### Total of B group vitamins: 1 to 10%

B group vitamins have a concept which includes all materials linked to vitamin B, and examples of B group vitamins include vitamin B1, vitamin B2, vitamin B6, niacin, pantothenic acid, biotin, vitamin B12, and folic acid. B group vitamins act as a co-enzyme of all enzymes. Being referred to as a metabolism enzyme, they generate body heat for sustaining human life or produce energy for muscle movement. Among them, vitamin B1 has an activity of promoting the power of generating heat by converting glucose in human body to energy, and vitamin B2 contributes to body temperature increase by adding body fat to energy. By containing those B group vitamins, the body temperature can be increased synergistically with lactoferrin. In addition to them, B group vitamins have an activity of absorbing slowly, in an intestinal tract, the lactoferrin arriving at intestine and propagating it evenly in cells of a human body, and thus they are a substance which function as a partner of lactoferrin. Furthermore, it has been proven to be difficult for B group vitamins to work if vitamin B1, vitamin B2, vitamin B6, niacin, pantothenic acid, biotin, vitamin B12, folic acid, and the like are individually ingested, and thus B group vitamins can work as a group in which those vitamins are mixed.

Furthermore, even though it is evident that B group vitamins are nutritional components which cannot be omitted for producing energy for sustaining human life, it is somewhat difficult to have them always without any omission based on common eating schedule. Due to such reasons, it is believed there are quite many people who are potentially deficient of B group vitamins. However, because B group vitamins are contained according to the present invention, there is also an advantage to have in balance the nutritional components that can be easily deficient.

When the total of B group vitamins is less than 1%, it is difficult to exhibit the aforementioned effect of increasing the body temperature, propagating lactoferrin evenly in cells of a human body, or the like. On the other hand, when the total of B group vitamins is more than 10%, the effect is saturated. Due to such reasons, the total of B group vitamins is at 1 to 10%. Furthermore, according to the present invention, it is not essential to contain B group vitamins.

### Blood circulation accelerator: 10 to 30%

The blood circulation accelerator is composed of a spice or a food material like Piper longum extract, ginger, hot pepper, pepper, and Zanthoxylum schinifolium which can accelerate blood circulation. The blood circulation accelerator can accelerate blood circulation and can enhance the body temperature increasing activity of lactoferrin. The blood circulation accelerator allows wide propagation of lactoferrin dissolved in blood even to the capillaries present at the terminal part of a human body. When the blood circulation accelerator is less than 10%, the lactoferrin's activity of increasing body temperature cannot be enhanced. On the other hand, when the blood circulation accelerator is more than 30%, the stimulation is excessively strong so that it becomes difficult for a patient to take it. Due to such reasons, the blood circulation accelerator is set at 10 to 30%.

Furthermore, for the remainder, a sweetening agent, an acidulant, a fragrance, or the like may be contained. Any known one can be applied for those sweetening agent, acidulant, and fragrance.

Furthermore, for the remainder, EPA (eicosapentaenoic acid) or nattokinase may be added. EPA or nattokinase has an effect of improving blood circulation as they allow smooth flow of blood. Furthermore, EPA or nattokinase has an activity of lowering neutral fat value and cholesterol value in blood and also an activity of maintaining blood in healthy state.

According to the aforementioned effects of EPA or nattokinase, poor blood circulation causing sensitivity to coldness can be improved and sensitivity to coldness itself can also be improved. As being contained at 0.3 to 3% for each, EPA or nattokinase can exhibit the aforementioned desired effect.

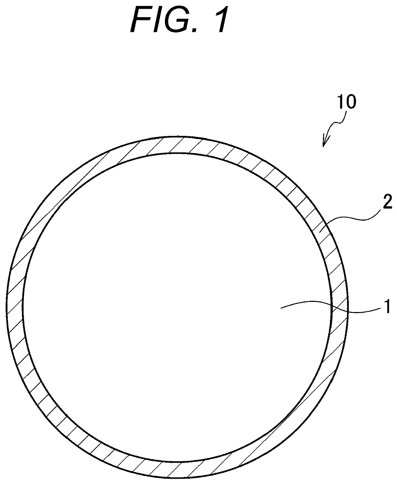

Furthermore, as shown in Fig. 1, it is also possible to have a tablet 10 having an auxiliary component 2 coated on a surface of a nucleating agent 1 containing a pharmaceutical component. When a surface of the nucleating agent 1 is coated with the auxiliary component 2, it is also possible that, as there is a part not completely coated with the auxiliary component 2, the nucleating agent 1 remains exposed. Namely, it is possible that at least part of the nucleating agent 1 is coated with the auxiliary component 2. Furthermore, other than the mode shown in Fig. 1, it is also possible to form one tablet by mixing an auxiliary component with a pharmaceutical component.

By holding this tablet in mouth, phosphoryl oligosaccharides of calcium and lactoferrin work in synergistic manner due to the presence of each other and saliva is secreted in a greater amount. As a result, dramatically increased secretion of saliva occurs in an oral cavity so that even a person with dry mouth symptom can easily intake a tablet with an aid of saliva.

Furthermore, according to an example, an application can also be made to a pharmaceutical product consisting of powder pharmaceutical or granular pharmaceutical mixed with an auxiliary component. Specifically, it is a pharmaceutical product obtained as a powder pharmaceutical which contains an auxiliary component in powder phase, said auxiliary component being formed with the aforementioned blending ratio, and a pharmaceutical component containing an effective component consisting of powder, or a granular pharmaceutical which contains an auxiliary component in granular phase and a pharmaceutical component containing an effective component consisting of granules. This powder pharmaceutical or granular pharmaceutical can also be those that are distributed, sold, and used after being sealed in a capsule. In addition, because the pharmaceutical component is the same as that of the aforementioned tablet, explanations are omitted hereinbelow by referring to those descriptions.

By holding this powder or granular pharmaceutical in mouth, phosphoryl oligosaccharides of calcium and lactoferrin work in synergistic manner due to the presence of each other and saliva is secreted in a greater amount. As a result, dramatically increased secretion of saliva occurs in an oral cavity so that even a person with dry mouth symptom can easily intake the powder or granular pharmaceutical with an aid of saliva.

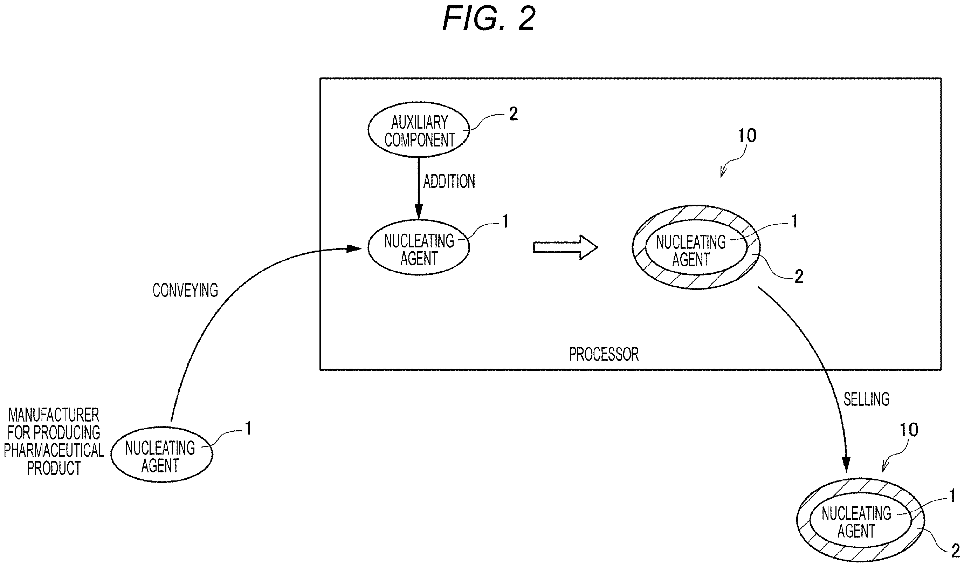

Next, explanations are given for the method for producing the pharmaceutical product with the constitution described above. Fig. 2 represents a flow of the method for producing a pharmaceutical product to which the present invention is applied.

First, the nucleating agent 1 containing the aforementioned pharmaceutical component is produced by a pharmaceutical product manufacturer. The nucleating agent can be in any form of a tablet, a liquid, a granule, a powder, or the like as long as it is a pharmaceutical component. In general, the nucleating agent 1 is distributed and sold in a market as it remains in the same state as it is produced by a pharmaceutical product manufacturer. However, according to the present invention, the nucleating agent 1 is acquired by a processor, and, after that, an operation of adding an auxiliary component is carried out.

In that case, the nucleating agent 1 is first acquired by a processor. Acquisition of the nucleating agent 1 can be made according to any method, but in any case, it is essential that, in a field in which the processing is actually carried out by a processor, the nucleating agent 1 is conveyed from an outside or an inside.

The auxiliary component 2 is added to the nucleating agent 1 by a processor. Addition of the auxiliary component 2 to the nucleating agent 1 can be carried out according to any mode. As for the addition method, it is possible that the nucleating agent 1 is simply admixed with an auxiliary component, or it is also possible that the nucleating agent 1 which has been conveyed is coated with the auxiliary component 2. Coating with the auxiliary component 2 may be carried out by a means like spraying with a spray or application onto the nucleating agent 1. Accordingly, a pharmaceutical product consisting of a tablet 10, which is obtained by adding the auxiliary component 2 to the nucleating agent 1, can be distributed and sold by a processor.

In addition, it is not limited to the flow shown in Fig. 2, and it is needless to say that an application can be made also for a case in which the pharmaceutical manufacturer and the processor are the same business operator. In that case, the nucleating agent 1 which has been produced in pharmaceutical production branch of the business operator is conveyed to processing branch, and in the processing branch, an operation of adding the auxiliary component 2 to the nucleating agent 1 is carried out as described above. Also in this case, a treatment is after all carried out for the nucleating agent 1 which has been conveyed. The part meant by this conveying is not limited to belt conveyer or the like, and it can be simply the performing of a treatment by drawing those stored in a storage place after they have been artificially transferred from other region.

Furthermore, regarding the step for adding the auxiliary component 2, it is possible that the auxiliary component 2 containing phosphoryl oligosaccharides of calcium and lactoferrin is added as a single body to the nucleating agent 1, or phosphoryl oligosaccharides of calcium and lactoferrin are separately added thereto.

Furthermore, regarding the step for adding the auxiliary component 2, it is preferable that the auxiliary component 2 is added such that it is contained at 0.01 to 50% in terms of % by mass relative to the total mass of a pharmaceutical product. However, it is not limited to this range and it can also be outside this range.

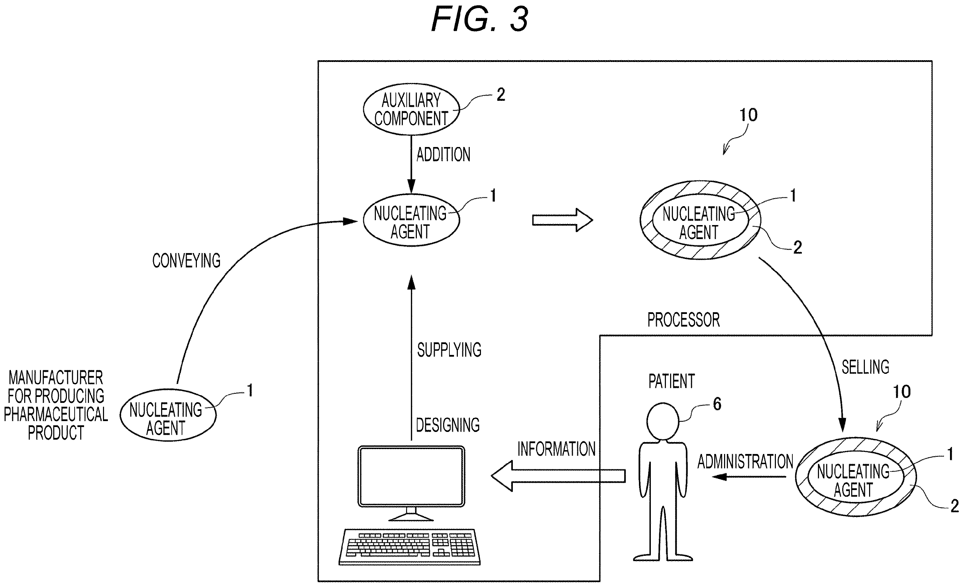

Furthermore, Fig. 3 shows an example in which the information regarding a patient 6, who is expected to have an administration, is collected and the information is reflected to designing of a pharmaceutical product. First, information is collected from the patient 6 who is expected to have an administration. With regard to the information to be collected, information regarding the saliva secretion property of the patient 6 can be collected in case of a tablet to be held in an oral cavity. In case of an application to an eye drop, information regarding tear secretion property from lacrimal gland can be collected. Furthermore, in case of an application to a nasal drop, information regarding the amount of snot or the like can be collected.

Next, based on the information which has been collected, a pharmaceutical product is actually designed. For the designing, in a case in which information regarding a saliva secretion property of a patient is collected, for example, the designing is made such that the content of phosphoryl oligosaccharides of calcium and lactoferrin varies depending on the saliva secretion property. For example, if the saliva secretion property is high, the necessity for promoting such saliva secretion via the auxiliary component 2 is not high, and thus the content of phosphoryl oligosaccharides of calcium and lactoferrin is set at a lower level. On the other hand, in a case in which the saliva secretion property is low and there is a dry mouth state, the content of phosphoryl oligosaccharides of calcium and lactoferrin is set at a higher level because the necessity for promoting the saliva secretion via the auxiliary component 2 is high. The information which has been set regarding the content of phosphoryl oligosaccharides of calcium and lactoferrin is sent to the step for adding the auxiliary component 2, and then utilized as the addition amount of the phosphoryl oligosaccharides of calcium, lactoferrin during that addition step. If the collected information is information regarding tear secretion amount from lacrimal gland instead of saliva secretion amount, the setting is made such that the addition amount of phosphoryl oligosaccharides of calcium and lactoferrin is either increased or decreased depending on that tear secretion amount.

By introducing the step for designing the content of phosphoryl oligosaccharides of calcium and lactoferrin as described above, it becomes possible to provide a pharmaceutical product which has been optimized to meet the needs of a patient.

In addition, a designing method is disclosed which consists at least of an information collecting step for collecting information from a patient as described above and a designing step for designing content of phosphoryl oligosaccharides of calcium and lactoferrin contained in an auxiliary component that is to be added based on the collected information. Namely, a consulting business for carrying out the aforementioned information collecting step and designing step as a main project is also included in the present invention.

In addition, it is also possible that setting is made during the designing step such that, as soon as the information of a patient is supplied to a personal computer (PC) by using a PC or the like, the optimum content is automatically generated through a design software installed in advance. This design software consists of a program which generates the content of phosphoryl oligosaccharides of calcium and lactoferrin as it is varied depending on the input information of a patient. For example, the order system is already set such that, for a case in which the saliva amount per minute is 30 cc or more, phosphoryl oligosaccharides of calcium is set at 5% and lactoferrin is set at 2%, and for a case in which the saliva amount per minute is less than 10 cc, phosphoryl oligosaccharides of calcium is set at 20% and lactoferrin is set at 8%, or the like. It is needless to say that the programming is made such that the upper limit and lower limit of the content of phosphoryl oligosaccharides of calcium and lactoferrin are within the aforementioned range.

Furthermore, for this designing step, the design can be made such that the auxiliary component is contained at 0.01 to 50% in terms of % by mass relative to the total mass of a pharmaceutical product, but it is not limited thereto. For example, when a pharmaceutical product is prepared with an auxiliary component only, the auxiliary component becomes 100% in terms of % by mass relative to the total mass of a pharmaceutical product, but it is obvious that this range is also included in said designing method.

Other than the method for producing a pharmaceutical product for treating a human body, said disclosure can also be applied to a method for producing a chemical composition. In this case, the chemical composition contains amain component for exhibiting a predetermined effect and an auxiliary component to be added to that main component. Hereinbelow, explanations are given for an example of a main component for constituting the chemical composition.

The chemical composition contains a main component and an auxiliary component. Details of the auxiliary component are the same as those described above. The auxiliary component is contained at 0.01 to 50% in terms of % by mass relative to the total mass of a chemical composition. If the auxiliary component is less than 0.01% in terms of % by mass relative to the total mass of a chemical composition, the aforementioned effect as an auxiliary component cannot be exhibited. On the other hand, if the auxiliary component is contained at more than 50% in terms of % by mass relative to the total mass of a chemical composition, the effect is saturated. Furthermore, it is also possible to prepare the chemical composition only with an auxiliary component.

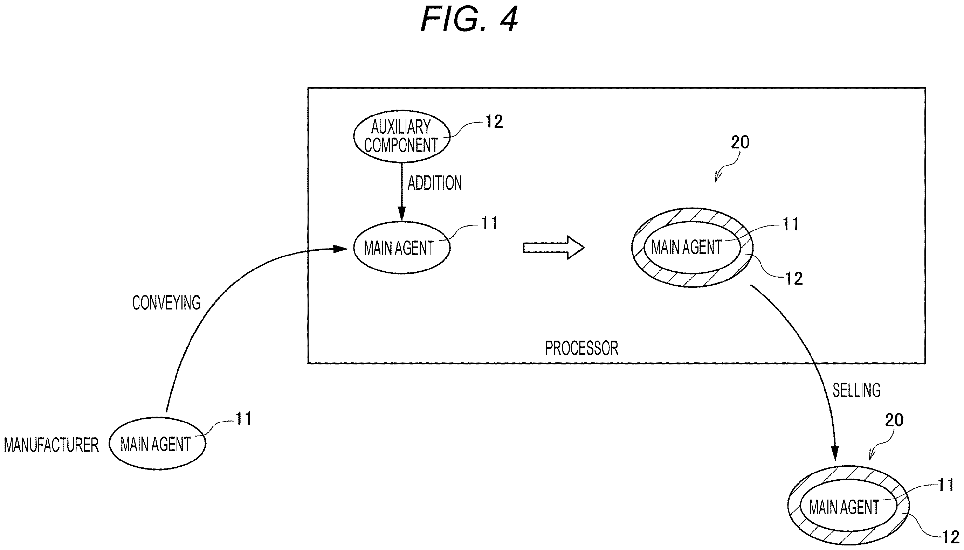

Next, the method for producing a chemical composition having the aforementioned constitution is explained. Fig. 4 represents a flow of the method for producing a chemical composition to which the present invention is applied.

First, a main agent 11 containing the aforementioned main component is produced by a manufacturer. The main agent 11 can be in any form of a tablet, a liquid, a granule, a powder, or the like. In general, the main agent 11 to be produced is distributed and sold in a market as it remains in the same state as it is produced. However, according to the present invention, the main agent 11 is acquired by a processor, and, after that, an operation of adding an auxiliary component is carried out.

In that case, the main agent 11 is first acquired by a processor. Acquisition of the main agent 11 can be made according to any method, but in any case, it is essential that, in a field in which the processing is actually carried out by a processor, the main agent 11 is conveyed from an outside or an inside.

The auxiliary component 12 is added to the main agent 11 by a processor. Addition of the auxiliary component 12 to the main agent 11 can be carried out according to any mode. As for the addition method, it is possible that the main agent 11 is simply admixed with an auxiliary component 12, or it is also possible that the main agent 11 which has been conveyed is coated with the auxiliary component 12. Coating with the auxiliary component 12 may be carried out by a means like spraying with a spray or application onto the main agent 11. Accordingly, a pharmaceutical product consisting of a chemical composition 20 which is obtained by adding the auxiliary component 12 to the main agent 11 can be distributed and sold by a processor.

In addition, it is not limited to the flow shown in Fig. 4, and it is needless to say that an application can be made also for a case in which the manufacturer and the processor are the same business operator. In that case, the main agent 11 which has been produced in production branch of the business operator is conveyed to processing branch, and in the processing branch, an operation of adding the auxiliary component 12 to the main agent 11 is carried out as described above. Also in this case, a treatment is after all carried out for the main agent 11 which has been conveyed. The part meant by this conveying is not limited to belt conveyer or the like, and it can be simply the performing of a treatment by drawing those stored in a storage place after they have been artificially transferred from other region.

Furthermore, regarding the step for adding the auxiliary component 12, it is possible that the auxiliary component 12 containing phosphoryl oligosaccharides of calcium and lactoferrin is added as a single body to the main agent 11, or phosphoryl oligosaccharides of calcium and lactoferrin are separately added thereto.

Furthermore, regarding the step for adding the auxiliary component 12, it is preferable that the auxiliary component 12 is added such that it is contained at 0.01 to 50% in terms of % by mass relative to the total mass of a chemical composition. However, it is not limited to this range and it can also be outside this range.

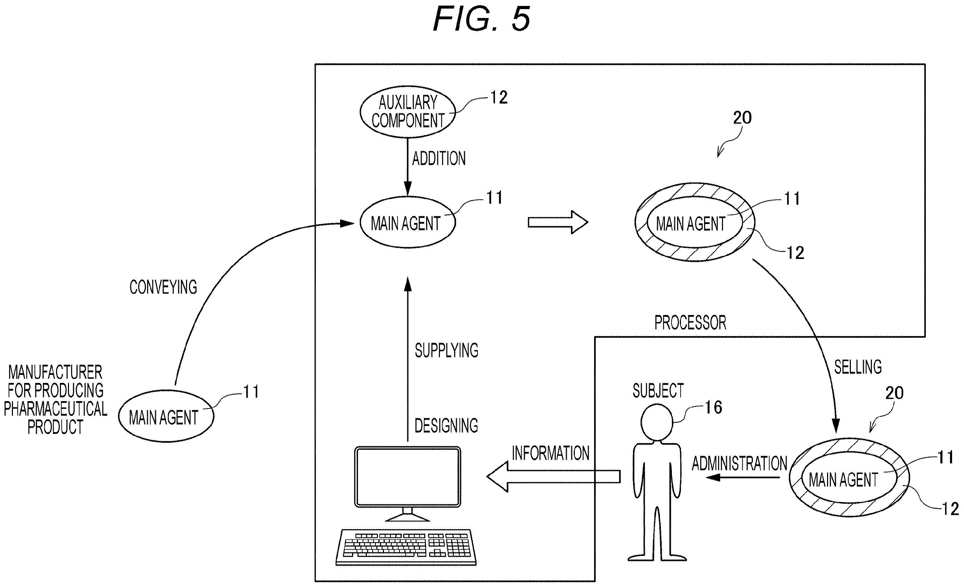

Furthermore, Fig. 5 represents an example in which information regarding a subject 16 to have a treatment is collected and reflected for designing of a chemical composition. First, information is collected from the subject 16 to have a treatment with a chemical composition. The treatment described herein includes an application of a chemical composition realized as a cosmetic product on skin, washing hair by using a chemical composition realized as hair cleansing agent, and the like. With regard to the information to be collected, information regarding the saliva secretion property of the patient 6 can be collected when the chemical composition is a food product. In case of an application to a hair cleansing agent, information regarding the amount or quality of sebum in hair can be collected.

Next, based on the information which has been collected, a chemical composition is actually designed. For the designing, in a case in which a saliva secretion property of the subject 16 is collected, for example, the designing is made such that the content of phosphoryl oligosaccharides of calcium and lactoferrin varies depending on the saliva secretion property. For example, if the saliva secretion property is high, the necessity for promoting such saliva secretion via the auxiliary component 12 is not high, and thus the content of phosphoryl oligosaccharides of calcium and lactoferrin is set at a lower level. On the other hand, in a case in which the saliva secretion property is low and there is a dry mouth state, the content of phosphoryl oligosaccharides of calcium and lactoferrin is set at a higher level because the necessity for promoting the saliva secretion via the auxiliary component 12 is high. The information which has been set regarding the content of phosphoryl oligosaccharides of calcium and lactoferrin is sent to the step for adding the auxiliary component 12, and then utilized as the addition amount of the phosphoryl oligosaccharides of calcium, lactoferrin during that addition step. If the collected information is information regarding sebum amount instead of saliva secretion amount, the setting is made such that the addition amount of phosphoryl oligosaccharides of calcium and lactoferrin is either increased or decreased depending on that sebum amount.

By introducing the step for designing the content of phosphoryl oligosaccharides of calcium and lactoferrin as described above, it becomes possible to provide a pharmaceutical product which has been optimized to meet the needs of a patient.

In addition, the disclosure can also be realized as a designing method which consists at least of an information collecting step for collecting information from a patient as described above and a designing step for designing content of phosphoryl oligosaccharides of calcium and lactoferrin contained in an auxiliary component that is to be added based on the collected information. Namely, a consulting business for carrying out the aforementioned information collecting step and designing step as a main project is also disclosed.

In addition, it is also possible that setting is made during the designing step such that, as soon as the information of a patient is supplied to a personal computer (PC) by using a PC or the like, the optimum content is automatically generated through a design software installed in advance. This design software consists of a program which generates the content of phosphoryl oligosaccharides of calcium and lactoferrin as it is varied depending on the input information of a patient. For example, the order system is already set such that, for a case in which the saliva amount per minute is 30 cc or more, phosphoryl oligosaccharides of calcium is set at 5% and lactoferrin is set at 2%, and for a case in which the saliva amount per minute is less than 10 cc, phosphoryl oligosaccharides of calcium is set at 20% and lactoferrin is set at 8%, or the like. It is needless to say that the programming is made such that the upper limit and lower limit of the content of phosphoryl oligosaccharides of calcium and lactoferrin are within the aforementioned range.

Furthermore, for this designing step, the design can be made such that the auxiliary component is contained at 0.01 to 50% in terms of % by mass relative to the total mass of a chemical composition, but it is not limited thereto. For example, when a chemical composition is prepared with an auxiliary component only, the auxiliary component becomes 100% in terms of % by mass relative to the total mass of a chemical composition, but it is obvious that this range is also included the designing method to which the present disclosure is applied.

Furthermore, according to the disclosure above, realization can be made as a treatment method by which a subject is treated with a chemical composition containing the aforementioned main component and an auxiliary component which contains phosphoryl oligosaccharides of calcium: 1 to 30% and lactoferrin: 0.01 to 10% in terms of % by mass relative to the total mass of the auxiliary component. Specific examples of this treatment are the same as those described above, but not limited thereto, and it is a concept including any activity as long as a subject is treated with the chemical composition consisting of the above blending components.

The method for producing a food and drink composition also follows the same flow as the method for producing a chemical composition shown in Fig. 4. A food material containing the aforementioned components for food and drink corresponds to the main agent 11, and after acquiring the main agent 11 by a processor, an operation of adding an auxiliary component is carried out. As for the details of the method for producing this food and drink composition, the explanations regarding the method for producing a chemical composition shown in Fig. 4 can be utilized, and thus further explanations are omitted hereinbelow.

Furthermore, collecting information of the subject 16, which corresponds to a consumer who eats and drinks the food and drink composition, and reflecting the information to design the food and drink composition are also actually carried out based on the same order as that of the flow shown in Fig. 5. First, information is collected from the subject 16 as a consumer who eats and drinks the food and drink composition. With regard to the information to be collected, information regarding the saliva secretion property of the subject 16 can be collected, or information regarding preference for a food product or a drink can be collected when the chemical composition is a food product.

A case in which the design of a food and drink composition is actually carried out based on collected information is also the same as the chemical composition case shown in Fig. 4 described above. As such, by referring to those explanations, further explanations are omitted hereinbelow.

In addition, the disclosure can also be realized as a designing method which consists at least of an information collecting step for collecting information from a patient as described above and a designing step for designing content of phosphoryl oligosaccharides of calcium and lactoferrin contained in an auxiliary component which is to be added based on the collected information. Namely, a consulting business for carrying out the aforementioned information collecting step and designing step as a main project is also included in the present disclosure.

In addition, it is also the same as the method for designing a chemical composition described above in that the setting is preferably made during the designing step such that, as soon as the information of a patient is supplied to a personal computer (PC) by using a PC or the like, the optimum content is automatically generated through a design software installed in advance.

Furthermore, for this designing step, the design can be made such that the auxiliary component is contained at 0.01 to 50% in terms of % by mass relative to the total mass of a chemical composition, but it is not limited thereto. For example, when a chemical composition is prepared with an auxiliary component only, the auxiliary component becomes 100% in terms of % by mass relative to the total mass of a chemical composition, but it is obvious that this range is also included the designing method to which the present invention is applied.

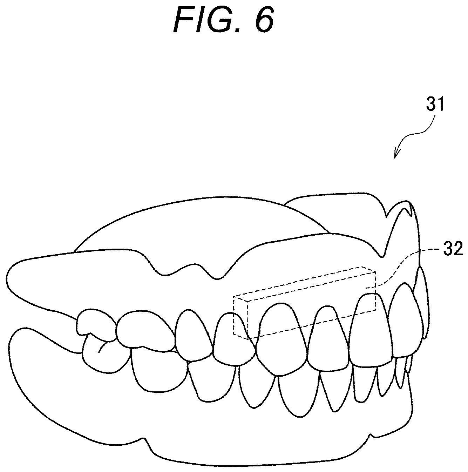

Fig. 6 shows an example of an oral fitting body 31 which is used for an actual application. The oral fitting body 31 is prepared as false teeth, a mouth piece, or the like which can be fitted in an oral cavity of a subject. On the inside of the oral fitting body 31, a charging pocket 32 is provided. In this charging pocket 32, an auxiliary component which contains phosphoryl oligosaccharides of calcium: 1 to 30% and lactoferrin: 0.01 to 10% in terms of % by mass relative to the total mass of the auxiliary component is charged. The auxiliary component is prepared in a liquid phase, a solid phase, a granular phase, a powder phase, or the like.

As the oral fitting body 31 is fitted in an oral cavity of a subject, the auxiliary component charged in the charging pocket 32 is dissolved and naturally diffused in the inside of an oral cavity of a subject. For reference, dissolution of the auxiliary component progresses with an aid of saliva or the temperature of an oral cavity of a subject.

As the auxiliary component naturally diffuses in the inside of an oral cavity of a subject, secretion of saliva is promoted. By having this constitution, the auxiliary component can be slowly dissolved in an oral cavity while a subject is sleeping during night time, in particular. As such, having a dry mouth state during sleeping can be prevented. Furthermore, when the oral fitting body 31 is fitted in a bed bound patient, administration of an auxiliary component can be easily achieved and also the 24-hour oral care can also be achieved.

### Example 1

Hereinbelow, the present invention is specifically explained in view of the test methods that are used in the present invention, and Examples and Comparative examples. However, the present invention is not limited to those Examples . Examples which, however, do not relate to oral care compositions as claimed, are also reference examples, such as nasal drops, cosmetic products which are not oral care compositions, and hair treatment products.

Furthermore, when % is indicated in the following examples, it is meant to represent % by mass.

In the present invention, the sample tablet which has been obtained for carrying out an experimental determination was held in the mouth of five people, i.e., the subject A to the subject E as shown in Tables 1 and 2, and then the saliva secretion amount was measured.

**[Table 1]**

| Constituents | Comparative example 1 | Comparative example 2 | Comparative example 3 | Comparative example 4 | Comparative example 5 | Comparative example 6 | Comparative. example 7 | Comparative example 8 | Comparative example 9 | Comparative example 10 | Present invention example 1 | Present invention example 2 | Present invention example 3 | Present invention example 4 |
|---|---|---|---|---|---|---|---|---|---|---|---|---|---|---|
| Sodium bicarbonate | 0 | 6 | 16 | | | | | | | | 16 | 16 | 16 | 16 |
| Phosphoryl oligosaccharides of calcium | | | | 2 | 4 | 10 | 20 | | | | 6 | 8 | 10 | 14 |
| Lactoferrin | | | | | | | | 0.2 | 0.4 | 1 | 1 | 1 | 1 | 1 |
| Potassium carbonate | | | | | | | | | | | | | | |
| Subject | | | | | | | | | | | | | | |
| A | 3 | 5 | 8 | 7 | 6 | 16 | 17 | 5 | 6 | 17 | 30 | 38 | 50 | 51 |
| B | 5 | 5 | 9 | 5 | 7 | 18 | 17 | 5 | 4 | 20 | 35 | 42 | 55 | 58 |
| C | 10 | 15 | 20 | 15 | 20 | 30 | 30 | 15 | 20 | 30 | 40 | 55 | 65 | 73 |
| D | 5 | 5 | B | 5 | 5 | 21 | 23 | 5 | 5 | 21 | 48 | 57 | 74 | 95 |
| E | 0 | 0 | 5 | 2 | 2 | 10 | 10 | 0 | 0 | 10 | 13 | 32 | 42 | 62 |
| Total saliva amount | 23 | 30 | 50 | 34 | 40 | 95 | 97 | 30 | 35 | 98 | 166 | 224 | 286 | 339 |

**[Table 2]**

| Constituents | Present invention example 5 | Present invention example 6 | Comparative example 11 | Present invention example 7 | Present invention example 8 | Present invention example 9 | Present invention example 10 | Present invention example 11 | Present invention example 12 | Present invention example 13 | Present invention example 14 | Present invention example 15 | Present invention example 16 |
|---|---|---|---|---|---|---|---|---|---|---|---|---|---|
| Sodium bicarbonate | 16 | 16 | 16 | 5 | 25 | 0 | 0 | 0 | 0 | 0 | 16 | | |
| Phosphoryl oligosaccharides of calcium | 16 | 20 | 35 | 16 | 16 | 16 | 2 | 6 | 16 | 25 | 20 | 6 | 6 |
| Lactoferrin | 1 | 1 | 1 | 1 | 1 | 1 | 0.05 | 4 | 6 | 9.5 | 1 | 4 | 4 |
| Potassium carbonate | | | | | | | | | | | 2 | | |
| Magnesium carbonate | | | | | | | | | | | | 3 | |
| Sucralose | | | | | | | | | | | | | 3 |
| | | | | | | | | | | | | | |
| Subject | | | | | | | | | | | | | |
| A | 56 | 56 | 55 | 45 | 55 | 42 | 14 | 48 | 57 | 73 | 59 | 63 | 68 |
| B | 63 | 60 | 61 | 51 | 61 | 41 | 16 | 54 | 62 | 86 | 64 | 63 | 61 |
| C | 81 | 83 | 82 | 65 | 78 | 50 | 24 | 67 | 84 | 92 | 88 | 76 | 81 |
| D | 116 | 118 | 114 | 84 | 112 | 71 | 34 | 81 | 119 | 103 | 122 | 97 | 99 |
| E | 78 | 75 | 76 | 51 | 68 | 43 | 21 | 45 | 67 | 81 | 79 | 68 | 75 |
| Total saliva amount | 394 | 392 | 388 | 296 | 374 | 247 | 109 | 295 | 389 | 435 | 412 | 367 | 384 |

The subject A is a man in his 50s, the subject B is a man in his 60s, the subject C is a man in his 40s, the subject D is a woman in her 50s, and the subject E is a woman in her 50s. The subject A to the subject E are all a person with dry mouth symptom. In particular, the subject E is a person who is under the treatment with an anti-cancer agent after the operation of tongue cancer and is still in a state of exhibiting generally almost no secretion of saliva.

Each of the subject A to the subject E was allowed to hold, for 1 minute, a sample tablet consisting of the components shown in Tables 1 and 2, and by having the saliva which has been pooled in the oral cavity ejected into a measuring cup, the amount of the saliva was measured. All the sample tablets were prepared as a tablet of 500 mg, and each component shown in Table 1 was contained at % with the numerical value given in the Table. The remainder consists of a starch component like dextrin.

The comparative example 1 is a so-called bulk state sample in which the sample tablet consists of dextrin only. In the comparative examples 2 and 3, the component for promoting the saliva secretion is sodium bicarbonate only, and the constitution was made such that content thereof is different from each other. In the comparative examples 4 to 7, the component for promoting the saliva secretion is phosphoryl oligosaccharides of calcium only, and the constitution was made such that content thereof is different from each other. In the comparative examples 8 to 10, the component for promoting the saliva secretion is lactoferrin only, and the constitution was made such that content thereof is different from each other.

In those comparative examples 2 to 10, there was a tendency that the saliva secretion amount increases in all of the subject A to the subject E as the content of the component is increased.

The present invention examples 1 to 14 are all within the range defined by the present invention. For example, in the present invention example 3, sodium bicarbonate is 16%, phosphoryl oligosaccharides of calcium is 10%, and lactoferrin is 1%. In the comparative example 3, however, only sodium bicarbonate is contained at 16%, and in the comparative example 6, only phosphoryl oligosaccharides of calcium is contained at 10%, and in the comparative example 10, only lactoferrin is contained at 1%. Also from the viewpoint that the saliva secretion amount in the present invention example 3 is more than the total saliva secretion amount of the comparative examples 3, 6, and 10, it is believed that a synergistic effect is exhibited by having a single composition according to mixing of those components.

Furthermore, the comparative example 11 and the present invention examples 1 to 6 show the result of measuring the saliva secretion amount from each while sodium bicarbonate is fixed at 16% and lactoferrin is fixed at 1% with variation of the content of phosphoryl oligosaccharides of calcium. According to the present invention examples 1 to 6, because phosphoryl oligosaccharides of calcium is contained within the range of 1 to 30%, total saliva secretion amount from 5 subjects was more than 150 ml. According to the comparative example 11, phosphoryl oligosaccharides of calcium is more than 30%, and thus the saliva secretion amount tends to be lower than the present invention examples 5 and 6. In addition, if phosphoryl oligosaccharides of calcium is more than 30%, a burden related to raw material cost becomes high. In addition to them, since several people among the subjects who have been administered with the comparative example 11 reported a symptom like an upset stomach or the like, the demerit of adding excessive amount of phosphoryl oligosaccharides of calcium was shown.

The present invention examples 9 to 13 are the examples in which the composition consists of phosphoryl oligosaccharides of calcium and lactoferrin only without addition of sodium bicarbonate. Also from the present invention examples 9 to 13, it is more than the total saliva secretion amount of the comparative examples 4 and 8, and thus it is believed that a synergistic effect is exhibited by having a single composition according to mixing of those components.

In the present invention example 8, content of phosphoryl oligosaccharides of calcium and lactoferrin is the same as that of the present invention example 7. However, the content of sodium bicarbonate is 25%, which is set to be beyond the range of 1 to 20%. It was shown that the saliva secretion amount can be further increased by enhancing the content of sodium bicarbonate. The saliva secretion amount can be increased accordingly in the present invention example 8. Furthermore, although there was no particular report made by this subject, one of the subjects treated with the present invention example 8 claims a symptom of minor nausea or the like, and thus the demerit of adding excessive amount of sodium bicarbonate was shown.

The present invention example 14 is an example in which potassium carbonate is additionally added at 2%. It was found that the saliva secretion amount can be dramatically increased by adding potassium carbonate.

The present invention example 15 is an example in which magnesium carbonate is contained at 3% in addition to the content of phosphoryl oligosaccharides of calcium and lactoferrin indicated for the present invention example 11. It was shown that the addition of magnesium carbonate in the present invention example 15 exhibits an increased saliva secretion amount.

Furthermore, the present invention example 16 is an example in which sucralose is contained at 3% in addition to the content of phosphoryl oligosaccharides of calcium and lactoferrin indicated for the present invention example 11. It was shown that the addition of sucralose in the present invention example 16 exhibits an increased saliva secretion amount.

Hereinbelow, explanations are given with regard to the specific examples of components.

An eye drop liquid containing an eye drop component and an auxiliary component was prepared. The auxiliary component constitutes 2.6% liquid in terms of % by mass relative to the total mass of the eye drop liquid. The auxiliary component consists of phosphoryl oligosaccharides of calcium: 16%, lactoferrin: 1% in terms of % by mass relative to the total mass of the auxiliary component, and water as remainder. Furthermore, like Sante Medical 10 (registered trademark) by Santen Pharmaceutical Co., Ltd., the eye drop component consists of, in terms of % by mass relative to the total mass of the eye drop component, vitamin B12 (cyanocobalamine): 0.02%, neostigmine methyl sulfate: 0.005%, vitamin B6 (pyridoxine hydrochloride) : 0.05%, panthenol: 0.05%, potassium L-asparaginic acid: 1.0%, taurine: 1.0%, chlorphenylamine maleate: 0.03%, epsilon-aminocapronic acid: 1.0%, dipotassium glycyrrhizic acid: 0.1%, and tetrahydrozoline hydrochloride: 0.03%. In addition to them, sodium edetate hydrate, chlorobutanol, benzalkonium chloride solution, boric acid, d-borneol, l-menthol, and a pH adjusting agent are added. Furthermore, the remainder of an eye drop is water or the like.

As a result of applying this eye drop component to a subj ect having a dry eye symptom, there was a response indicating that the dry eye symptom is improved compared to before.

Furthermore, the same subject was tested with a case in which the auxiliary component consists of phosphoryl oligosaccharides of calcium: 16%, lactoferrin: 1% in terms of % by mass relative to the total mass of the auxiliary component, and water as remainder, and the auxiliary component constitutes 2.6% liquid in terms of % by mass relative to the total mass of the eye drop liquid while the eye drop component consists of water only. Similar to the above, there was a response indicating that the dry eye symptom is improved compared to before.

A nasal drop liquid containing a nasal drop component and an auxiliary component was prepared. The auxiliary component constitutes 3.6% liquid in terms of % by mass relative to the total mass of the eye drop liquid. The auxiliary component consists of phosphoryl oligosaccharides of calcium: 6%, lactoferrin: 2.5% in terms of % by mass relative to the total mass of the auxiliary component, and water as remainder. Furthermore, similar to Shin Lulu (registered trademark) nasal drop, the nasal drop component consists of 0.5 mg of naphazoline hydrochloride, 5 mg of chlorphenylamine maleate, 3 mg of lidocaine, and 0.2 mg of benzetonium hydrochloride in 1 ml, and it is obtained by adding an isotonic agent, paraben, and a pH adjusting agent.

As a result of applying this nasal drop component to a subject, there was a response indicating that the motion stability suggesting an action on parasympathetic neurons is shown and, based on the therapeutic effect of having wider vision due to an improvement of a dry eye, symptoms considered to be related to the activity of improved brain function are recovered compared to before.

Furthermore, the same subject was tested with a case in which the auxiliary component consists of phosphoryl oligosaccharides of calcium: 6%, lactoferrin: 2.5% in terms of % by mass relative to the total mass of the auxiliary component, and water as remainder, and the auxiliary component constitutes 3.6% liquid in terms of % by mass relative to the total mass of the nasal drop liquid while the nasal drop component consists of water only. Similar to the above, there was a response indicating that the dry eye symptom, wider vision, or the like are improved compared to before.

Furthermore, a nasal drop liquid containing a nasal drop component consisting of Nazal (registered trademark, trade name) manufactured by Sato Pharmaceutical Co., Ltd. and an auxiliary component was prepared. The auxiliary component constitutes 3.6% liquid in terms of % by mass relative to the total mass of the eye drop liquid. The auxiliary component consists of phosphoryl oligosaccharides of calcium: 6%, lactoferrin: 2.5% in terms of % by mass relative to the total mass of the auxiliary component, and water as remainder. As a result of applying it to a subject, the same response as above was obtained.

A cosmetic product containing a cosmetic component and an auxiliary component was prepared. The auxiliary component constitutes 5.2% liquid in terms of % by mass relative to the total mass of the cosmetic product. The auxiliary component consists of phosphoryl oligosaccharides of calcium: 12%, lactoferrin: 1% in terms of % by mass relative to the total mass of the auxiliary component, and water as remainder. Furthermore, the cosmetic component is obtained by adding, in terms of % by mass relative to the total mass of the cosmetic component, a whitening component (L-ascorbic acid 2-glucoside or the like) : 2.00%, a component for preventing skin roughness (glycyrrhizic acid2K) : 0.10%, a moisturizing component (conc. glycerin: 8.00%, trehalose solution: 1.00%, sodium hyaluronate: 0.05%, oily emollient) component (soy bean oil) : 0.03%, pH adjusting agent: 0.03%, a thickening agent (xanthan gum: 0.30%, hydroxyethyl cellulose: 0.30%), a solubilizing agent (polyoxyethylene hydrogenated castor oil) : 0.5%, a preservative (methylparaben: 0.21%, phenoxy ethanol: 0.11%), a chelating agent (EDTA-2Na): 0.10%, natural vitamin E: 0.07%, and remainder: purified water.

As a result of applying this cosmetic product to a subject, there was a response indicating that symptoms of skin roughness, skin darkness, and dry skin are recovered compared to before.

Furthermore, the same subject was tested with a case in which the auxiliary component consists of phosphoryl oligosaccharides of calcium: 12%, lactoferrin: 1% in terms of % by mass relative to the total mass of the auxiliary component, and water as remainder, and the auxiliary component constitutes 5.2% liquid in terms of % by mass relative to the total mass of the nasal drop liquid while the cosmetic component consists of water only. Similar to the above, there was a response indicating that symptoms of skin roughness and skin darkness are recovered compared to before.

Furthermore, a cosmetic product containing a cosmetic component consisting of Gokujun (registered trademark, trade name) manufactured by Rohto Pharmaceutical Co., Ltd. and an auxiliary component was prepared. The auxiliary component constitutes 5.2% liquid in terms of % by mass relative to the total mass of the cosmetic product. The auxiliary component consists of phosphoryl oligosaccharides of calcium: 12%, lactoferrin: 1% in terms of % by mass relative to the total mass of the auxiliary component, and water as remainder. As a result of applying the resulting cosmetic product to a subject, there was a response indicating that symptoms of skin roughness, skin darkness, and dry skin are recovered compared to before.

A tablet containing a pharmaceutical component and an auxiliary component was prepared. The auxiliary component constitutes 2.3% in terms of % by mass relative to the total mass of the tablet, and a surface of the pharmaceutical component is coated by it. The auxiliary component consists of phosphoryl oligosaccharides of calcium: 7%, lactoferrin: 3% in terms of % by mass relative to the total mass of the auxiliary component, and starch as remainder. Furthermore, the pharmaceutical component is obtained by containing, in terms of % by mass relative to the total mass of the pharmaceutical component, takadiastase N1: 150 mg, lipase AP12: 60 mg, an extract of mallotus japonicus: 63 mg, licorice powder: 150 mg, magnesium silicate aluminate: 720 mg, synthetic hydrotalcite: 300 mg, magnesium hydroxide: 600 mg, an extract of scopolia: 30 mg, amur cork tree powder: 105 mg, cinnamon powder: 225 mg, fennel powder: 60 mg, clove powder: 30 mg, ginger powder: 75 mg, and I-menthol: 9 mg in 9 tablets. As a result of having this tablet taken by a subject, there was a response indicating that not only the stomach state is improved but also the saliva secretion amount can be increased when it is held in a mouth and the tablet can be more easily taken.

Similar to the above, powder and granules were prepared with the same components as the above tablet, and as a result of applying them to a subject, the same response as above was obtained.

A hair cleansing composition (i.e., shampoo) containing a cleansing component and an auxiliary component was prepared. The auxiliary component constitutes 5.6% in terms of % by mass relative to the total mass of the shampoo. The auxiliary component consists of phosphoryl oligosaccharides of calcium: 12%, lactoferrin: 2% in terms of % by mass relative to the total mass of the auxiliary component, and water as remainder. Furthermore, the cleansing component is obtained by containing, in terms of % by mass relative to the total mass of the cleansing component, water: 53.3%, a surface active agent (i.e., soap): 42.0%, a foaming agent: 3.0%, a fragrance: 0.5%, a preservative: less than 0.5%, a pH adjusting agent: 0.5%, and a conditioning agent: 0.5%. As a result of using this shampoo for hair washing by a subject, there was a response indicating that dandruff is improved or glossy hair is obtained.

A hair treatment product (i.e., rinse) containing a hair treatment component and an auxiliary component was prepared. The auxiliary component constitutes 7.1% in terms of % by mass relative to the total mass of the rinse. The auxiliary component consists of phosphoryl oligosaccharides of calcium: 12%, lactoferrin: 2% in terms of % by mass relative to the total mass of the auxiliary component, and water as remainder. Furthermore, the hair treatment component is constituted with, in terms of % by mass relative to the total mass of the hair treatment component, myristyl alcohol: 5.00%, dimethicone: 1.00%, ethyl hexyl isononanoic acid: 1.00%, (stearoxymethicone/dimethicone) copolymer: 0.50%, hydrogenated olive oil: 0.50%, diisobutyl adipic acid: 0.25%, diisopropyladipicacid: 0.25%, lavender oil: 0.03%, glycerin: 5.00%, glycine: 1.00%, di(cholesteryl/octyldodecyl) lauroyl glutamic acid: 0.20%, ethyl diethylaminestearamide: 3.00%, ethyl cocolyarginine PCA: 0.20%, hydrolyzed silk: 0.01%, glycolic acid: 0.63%, hydrogenated lecithine: 0.50%, ethanol: 0.01%, and water as remainder. As a result of using this rinse for hair washing by a subject, there was a response indicating that dandruff is improved or glossy hair is obtained.

A jelly snack containing a food component and an auxiliary component was prepared. The auxiliary component constitutes 3.1% in terms of % by mass relative to the total mass of the snack. The auxiliary component consists of phosphoryl oligosaccharides of calcium: 16%, lactoferrin: 6% in terms of % by mass relative to the total mass of the auxiliary component, and water as remainder. Furthermore, the food component consists of 82.3 g of water, 2.3 g of protein, 15.3 g of carbohydrate, 0.1 g of fiber, and 3 mg of calcium in 100 g. As a result of allowing a subject to have this jelly snack, there was a response indicating that saliva is secreted in large quantities.

A drink composition containing a drink component which consists of "Southern Alps natural water" (registered trademark) manufactured by Suntory Holdings Limited and an auxiliary component was prepared. The auxiliary component constitutes 10% in terms of % by mass relative to the total mass of the drink composition. The auxiliary component consists of phosphoryl oligosaccharides of calcium: 12%, lactoferrin: 4% in terms of % by mass relative to the total mass of the auxiliary component, and water as remainder. Furthermore, the drink component was "Southern Alps natural water" (registered trademark) manufactured by Suntory Holdings Limited having sodium: 0.4 to 1.0 mg, calcium: 0.6 to 1.5 mg, magnesium: 0.1 to 0.3 mg, potassium: 0.1 to 0.5 mg, hardness: 30, and pH: about 7. As a result of allowing a subject to have this drink, there was a response indicating that saliva is secreted in large quantities.

A seasoning containing a seasoning component which consists of soy sauce manufactured by Kikkoman Corporation and an auxiliary component was prepared. The auxiliary component constitutes 5% in terms of % by mass relative to the total mass of the seasoning. The auxiliary component consists of phosphoryl oligosaccharides of calcium: 12%, lactoferrin: 4% in terms of % by mass relative to the total mass of the auxiliary component, and water as remainder. Furthermore, the seasoning component was soy sauce manufactured by Kikkoman Corporation. As a result of allowing a subject to use this seasoning, there was a response indicating that smoother taste is obtained.

### Example 2

Hereinbelow, the present invention is specifically explained in view of the test methods that are used in the present invention, and Examples and Comparative examples. However, the present invention is not limited to those Examples. Furthermore, when it is simply described with % in the following examples, it is meant to represent % by mass.

In the present invention, a sample tablet which has been obtained for carrying out an experimental determination was held in the mouth of five people, i.e., the subject A to the subject E, as shown in Tables 3 and 4, and then the saliva secretion amount was measured.

**[Table 3]**

| Constituents | Comparative example 1 | Comparative example 2 | Comparative example 3 | Comparative example 4 | Comparative example 5 | Comparative example 6 | Comparative example 7 | Comparative example 8 | Comparative example 9 | Comparative example 10 | Comparative example 11 | Present invention example 17 | Present invention example 18 |
|---|---|---|---|---|---|---|---|---|---|---|---|---|---|
| Sodium bicarbonate | 0 | 6 | 16 | | | | | | | | 0 | 0 | 0 |
| Phosphoryl oligosaccharides of calcium | | | | 2 | 4 | 10 | 20 | | | | 0.6 | 3 | 2 |
| Lactoferrin | | | | | | | | 0.2 | 0.4 | 1 | 6 | 6 | 2.5 |
| Potassium carbonate | | | | | | | | | | | | | |
| Subject | | | | | | | | | | | | | |
| A | 3 | 5 | 8 | 7 | 6 | 16 | 17 | 5 | 6 | 17 | 21 | 45 | 41 |
| B | 5 | 5 | 9 | 5 | 7 | 18 | 17 | 5 | 4 | 20 | 34 | 61 | 50 |
| C | 10 | 15 | 20 | 15 | 20 | 30 | 30 | 15 | 20 | 30 | 41 | 68 | 52 |
| D | 5 | 5 | 8 | 5 | 5 | 21 | 23 | 5 | 5 | 21 | 20 | 87 | 68 |
| E | 0 | 0 | 5 | 2 | 2 | 10 | 10 | 0 | 0 | 10 | 15 | 51 | 52 |
| Total saliva amount | 23 | 30 | 50 | 34 | 40 | 95 | 97 | 30 | 35 | 98 | 131 | 312 | 263 |

**[Table 4]**

| Constituents | Present invention example 19 | Present invention example 20 | Present invention example 21 | Present invention example 22 | Present invention example 23 | Present invention example 24 | Present invention example 25 | Present invention example 26 | Comparative example 12 | Present invention example 28 | Present invention example 29 | Comparative example 13 |
|---|---|---|---|---|---|---|---|---|---|---|---|---|
| Sodium bicarbonate | 0 | 0 | 0 | 0 | 0 | 16 | 8 | 16 | 0 | 0 | 10 | 0 |
| Phosphoryl oligosaccharides of calcium | 3.8 | 3.9 | 10 | 25 | 20 | 3 | 20 | 3 | 6 | 3 | 3 | 3 |
| Lactoferrin | 0.6 | 9.5 | 4 | 9.5 | 0.1 | 6 | 0.1 | 6 | 12 | 0.1 | 0.1 | 0.005 |
| Potassium carbonate | | | | | | | | 2 | | | | |
| Subject | | | | | | | | | | | | |
| A | 38 | 62 | 65 | 73 | 42 | 71 | 58 | 82 | 62 | 34 | 43 | 13 |
| B | 41 | 60 | 65 | 86 | 46 | 68 | 61 | 75 | 54 | 35 | 41 | 8 |
| C | 54 | 83 | 81 | 92 | 54 | 92 | 85 | 98 | 83 | 45 | 62 | 22 |
| D | 62 | 91 | 90 | 103 | 73 | 90 | 92 | 105 | 81 | 43 | 75 | 35 |
| E | 39 | 68 | 62 | 81 | 54 | 62 | 59 | 71 | 57 | 26 | 42 | 21 |
| Total saliva amount | 234 | 364 | 363 | 435 | 269 | 383 | 355 | 431 | 337 | 183 | 263 | 99 |

The subject A is a man in his 50s, the subject B is a man in his 60s, the subject C is a man in his 40s, the subject D is a woman in her 50s, and the subject E is a woman in her 50s. The subject A to the subject E are all a person with dry mouth symptom. In particular, the subject E is a person who is under the treatment with an anti-cancer agent after the operation of tongue cancer and is still in a state of exhibiting generally almost no secretion of saliva.

Each of the subject A to the subject E was allowed to hold, for 1 minute, a sample tablet consisting of the components shown in Tables 3 and 4, and by having the saliva which has been pooled in the oral cavity ejected into a measuring cup, the amount of the saliva was measured. All the sample tablets were prepared as a tablet of 500 mg, and each component shown in Tables 1 and 2 was contained at % with the numerical value given in the Tables. The remainder consists of a starch component like dextrin.

For the comparative examples 1 to 10 of Table 3, those that are the same as the comparative examples 1 to 10 of Table 1 are used. The comparative example 11 is an example in which the content of phosphoryl oligosaccharides of calcium is less than 1% as a lower limit, but is was shown that the saliva secretion amount did not increase that much. The comparative example 13 is an example in which the content of lactoferrin is less than 0.01% as a lower limit.

The present invention examples 17 to 20, 28, and 29 are all an example containing phosphoryl oligosaccharides of calcium: 1% to less than 4% and lactoferrin: 0.01% to 10%. Compared to the comparative examples 2 to 11 and 13, all showed an increased saliva secretion amount. Furthermore, the present invention examples 5 to 9 are all an example containing phosphoryl oligosaccharides of calcium: 4% to 30% and lactoferrin: 0.01% to less than 0.2%, or more than 3% to 10%. However, compared to the comparative examples 2 to 11 and 13, all showed an increased saliva secretion amount.

Incidentally, because the content of phosphoryl oligosaccharides of calcium is 12% in the comparative example 12, it was found the effect is saturated. In addition, from the viewpoint that the content of phosphoryl oligosaccharides of calcium is high, an increase in raw material cost is caused accordingly.

The present invention examples 24, 25, and 29 are an example in which sodium bicarbonate is contained in addition to the phosphoryl oligosaccharides of calcium and lactoferrin that are contained within the range defined by the present invention, and it was found that the saliva secretion amount has increased more. Furthermore, the present invention example 26 is an example in which potassium carbonate is additionally added at 2%. It was found that the saliva secretion amount can be dramatically increased by addition of potassium carbonate.

### Example 3

A subj ect was asked to hold in his/her mouth a sample tablet which has been obtained for carrying out an experimental determination regarding a tooth pain relief agent, and then the tooth pain relief effect was determined via an interview.

The sample tablet was prepared by mixing the auxiliary component shown in the following Table 5 with a main component. The numerical values in Table 5 represent % by mass relative to total mass.

**[Table 5]**

| | Comparative example 14 | Comparative example 15 | Present invention example 30 | Present invention example 31 | Present invention example 32 | Present invention example 33 | Present invention example 34 | Present invention example 35 | Comparative example 16 |
|---|---|---|---|---|---|---|---|---|---|
| Phosphoryl oligosaccharides of calcium | 4 | | 6 | 16 | 25 | 16 | 16 | 16 | 16 |
| Lactoferrin | | 6 | 1 | 4 | 9 | 4 | 4 | 4 | 4 |
| Lactoperoxidase | | | | | | 2 | 10 | 18 | 24 |
| Tooth pain relieving effect | 1 | 1 | 3 | 4 | 4 | 5 | 5 | 5 | 4 |

The subject was a man in his 50s, who has been diagnosed with C3 according to examination by a dentist as dental caries of the lower first premolar progressed to the dental pulp. Furthermore, as a result of interviewing the subject before the test, it was confirmed that the subject has a subjective symptom relating to tooth pain.

The subject was asked to hold strongly the sample tablet for 10 minutes by using his lower first premolar with dental caries. Then, he was allowed to evaluate the tooth pain relief effect based on 5 levels. As the relief effect is close to 5 in the table, the relief effect is higher, and thus it was determined that there is better stabilization of tooth pain. On the other hand, as the relief effect is close to 1 in the table, the relief effect is smaller, and thus it was determined that there is almost no stabilization of tooth pain.

All the sample tablets were prepared as a tablet of 500 mg, and each component shown in each table was contained only at % with the numerical value given in the table. The remainder consists of a starch component like dextrin.

In the comparative example 14, the sample tablet is simply prepared with phosphoryl oligosaccharides of calcium only. However, the tooth pain relief effect was small. Similarly, in the comparative example 15, the sample tablet is simply prepared with lactoferrin only, and the tooth pain relief effect was small.

On the other hand, the present invention examples 30 to 32 are an example in which phosphoryl oligosaccharides of calcium and lactoferrin are contained within the range defined by the present invention, and a high tooth pain relief effect was reported by the subject.

Furthermore, the present invention examples 33 to 35 are an example in which lactoperoxidase is contained within the range defined by the present invention in addition to the phosphoryl oligosaccharides of calcium and lactoferrin that are contained within the range defined by the present invention, and a high tooth pain relief effect was reported by the subject. Meanwhile, according to the comparative example 16 in which the content of lactoperoxidase is more than 20%, the effect appears to be slightly saturated.

### Example 4

Hereinbelow, explanations are given for an example of an agent for improving low body temperature to which the present invention is applied. Furthermore, when it is simply described with % in the following examples, it is meant to represent % by mass.

In the present invention, a sample consisting of each component shown in Table 6 and 7 was prepared for carrying out an experimental determination. After preparing the sample as granules, five people of the subject A to the subject E were asked to take it after dissolving in water. Then, the body temperature increase amount (hereinbelow, described as increased body temperature (°C)) was measured. As for the components for constituting each sample of this test, Piper longum extract was used as a blood circulation accelerator in addition to lactoferrin, phosphoryl oligosaccharides of calcium, and magnesium sulfate. Furthermore, the content of each component constituting B group vitamins and their total content are also shown. An example additionally containing EPA and nattokinase is also shown. All the numerical values given in the table indicate % by mass relative to the total mass of the auxiliary component. For measuring the increased body temperature, the body temperature before administration of a sample was measured first, and 60 minutes to 90 minutes after the administration of the sample, the body temperature was measured. In addition, the difference between the body temperature measured before the administration of the sample and the body temperature measured after the administration of the sample was used as an increased body temperature (°C).

**[Table 6]**

| | | | Comparative example | Comparative example | Comparative example | Comparative example | Comparative example | Comparative example | Comparative example | Comparative example | Comparative example | Comparative example | Present invention example | Present invention example | Present invention example | Comparative example | Comparative example | Present invention example | Comparative example |
|---|---|---|---|---|---|---|---|---|---|---|---|---|---|---|---|---|---|---|---|
| Sample number | | | 17 | 18 | 19 | 20 | 21 | 22 | 23 | 24 | 25 | 26 | 36 | 37 | 38 | 27 | 28 | 39 | 29 |
| | Lactoferrin | | | | 8 | 13 | 0.2 | 5 | 8 | 0 | 12 | 0.2 | 5 | 5 | 5 | 9 | 8 | 8 | 8 |
| | Phosphoryl oligosaccharides of calcium | | 4 | 10 | | | 2 | 8 | 14 | 1 | 2 | 0.5 | 8 | 8 | 8 | 33 | 22 | 22 | 12 |
| | Magnesium sulfate | | | | | | | | | 4 | 38 | 3 | 20 | 35 | 68 | 45 | 0.5 | 24 | 77 |
| | Zinc-containing yeast | | | | | | | | | | | | | | | | | | |
| | Piper longum extract | | | | | | | | | | | | | | | | | | |
| Component | | Total of B group vitamins | | | | | | | | | | | | | | | | | |
| | | Vitamin B1 | | | | | | | | | | | | | | | | | |
| | | Vitamin B2 | | | | | | | | | | | | | | | | | |
| | B group vitamins | Vitamin B6 | | | | | | | | | | | | | | | | | |
| | | Vitamin B12 | | | | | | | | | | | | | | | | | |
| | | Ca pantothenate | | | | | | | | | | | | | | | | | |
| | | Niacin | | | | | | | | | | | | | | | | | |
| | | Folic acid | | | | | | | | | | | | | | | | | |
| | Subject | | | | | | | | | | | | | | | | | | |
| | A | | 0 | 0 | 0 | 0 | 0 | 0 | 0 | 0 | 0.6 | 0.1 | 0.7 | 0.5 | 0.7 | 0.3 | 0.1 | 0.8 | 0.8 |
| Increased body temperature (°C) | B | | 0 | 0 | 0.1 | 0 | 0.1 | 0.2 | 0.2 | 0 | 0.5 | 0 | 0.6 | 0.7 | 0.5 | 0.4 | 0.2 | 0.7 | 0.7 |
| | C | | 0 | 0 | 0 | 0 | 0 | 0 | 0 | 0 | 0.4 | 0.2 | 0.5 | 0.4 | 0.5 | 0.4 | 0.2 | 0.5 | 0.8 |
| | D | | 0 | 0 | 0 | 0 | 0.1 | 0 | 0 | 0 | 0.4 | 0 | 0.6 | 0.5 | 0.7 | 0.4 | 0.1 | 0.7 | 0.6 |
| | E | | 0 | 0 | 0 | 0 | 0 | 0.1 | 0 | 0 | 0.3 | 0.1 | 0.4 | 0.3 | 0.5 | 0.2 | 0 | 0.5 | 0.4 |
| | Total | | 0 | 0 | 0.1 | 0 | 0.2 | 0.3 | 0.2 | 0 | 2.2 | 0.4 | 2.8 | 2.4 | 2.9 | 1.7 | 0.6 | 3.2 | 3.3 |

**[Table 7]**

| | | | Present invention example | Present invention example | Present invention example | Present invention example | Present invention example | Present invention example | Present invention example | Present invention example | Present invention example | Present Invention example | Present invention example | Present invention example | Present invention example | Present invention example | Present invention example |
|---|---|---|---|---|---|---|---|---|---|---|---|---|---|---|---|---|---|
| Sample number | | | 40 | 41 | 42 | 43 | 44 | 45 | 46 | 47 | 48 | 49 | 50 | 51 | 52 | 53 | 54 |
| Component | Lactoferrin | | 8 | 8 | 8 | 8 | 8 | 8 | 8 | 8 | 8 | 8 | 7 | 8 | 7 | 8 | 8 |
| | Phosphoryl oligosaccharides of calcium | | 10 | 10 | 10 | 10 | 10 | 10 | 10 | 10 | 10 | 10 | 10 | 10 | 9 | 10 | 10 |
| | Magnesium sulfate | | 37 | 37 | 37 | 37 | 37 | 37 | 37 | 37 | 37 | 37 | 35 | | | 37 | 37 |
| | Zinc-containing yeast | | | | | | | | | | | | | | | | |
| | Piper longum extract | | | | | | | 7 | 12 | 22 | 29 | 32 | 15 | 15 | | | |
| | B group vitamins | Total of B group vitamins | 0.5 | 1.5 | 5 | 9 | 11 | | | | | | 5 | | 4 | | |
| | | Vitamin B1 | 0.075 | 0.224 | 0.748 | 1.347 | 1.646 | | | | | | 0.748 | | 0.599 | | |
| | | Vitamin B2 | 0.058 | 0.175 | 0.582 | 1.047 | 1.280 | | | | | | 0.582 | | 0.465 | | |
| | | Vitamin B6 | 0.075 | 0.224 | 0.748 | 1.347 | 1.646 | | | | | | 0.748 | | 0.599 | | |
| | | Vitamin B12 | 0.021 | 0.063 | 0.210 | 0.379 | 0.463 | | | | | | 0.210 | | 0.168 | | |
| | | Ca pantothenate | 0.035 | 0.105 | 0.351 | 0.632 | 0.773 | | | | | | 0.351 | | 0.281 | | |
| | | Niacin | 0.232 | 0.697 | 2.323 | 4.182 | 5.112 | | | | | | 2.323 | | 1.859 | | |
| | | Folic acid | 0.004 | 0,011 | 0.037 | 0.066 | 0.080 | | | | | | 0.037 | | 0.029 | | |
| | EPA | | | | | | | | | | | | | | | 0.74 | |
| | Nattokinase | | | | | | | | | | | | | | | | 0.75 |
| Increased body temperature (°C) | Subject | | | | | | | | | | | | | | | | |
| | A | | 0.6 | 0.8 | 0.7 | 0.8 | 0.5 | 0.7 | 0.8 | 0.7 | 0.8 | 0.7 | 0.9 | 0.6 | 0.5 | 0.6 | 0.6 |
| | B | | 0.7 | 0.7 | 0.8 | 0.8 | 0.6 | 0.5 | 0.7 | 0.6 | 0.7 | 0.6 | 0.9 | 0.5 | 0.6 | 0.6 | 0.7 |
| | C | | 0.6 | 0.7 | 0.8 | 0.7 | 0.6 | 0.6 | 0.7 | 0.6 | 0.7 | 0.8 | 0.7 | 0.5 | 0.4 | 0.5 | 0.6 |
| | D | | 0.6 | 0.8 | 0.7 | 0.6 | 0.7 | 0.4 | 0.7 | 0.6 | 0.5 | 0.6 | 0.7 | 0.4 | 0.5 | 0.4 | 0.5 |
| | E | | 0.4 | 0.5 | 0.5 | 0.4 | 0.5 | 0.4 | 0.7 | 0.5 | 0.5 | 0.6 | 0.7 | 0.3 | 0.4 | 0.5 | 0.4 |
| | Total | | 2.9 | 3.5 | 3.5 | 3.3 | 2.9 | 2.6 | 3.6 | 3 | 3.2 | 3.3 | 3.9 | 2.3 | 2.4 | 2.6 | 2.8 |

The subject A is a man in his 40s, the subject B is a woman in her 60s, the subject C is a man in his 40s, the subject D is a woman in her 40s, and the subject E is a woman in her 50s. The subject A to the subject E all suffer from a symptom related to sensitivity to coldness.

For each of the subject A to the subject E, an agent for improving low body temperature containing an auxiliary component, which consists of the components shown in the above Tables 6 and 7, were prepared as granules (i.e., the present invention examples 36 to 54 and the comparative examples 17 to 29). Remainder of the auxiliary component consists of zinc-containing yeast, a bulking agent, an acidulant, a sweetening agent, or the like. The auxiliary component contained in this agent for improving low body temperature is 38.48% in terms of % by mass relative to the total mass of the agent for improving low body temperature. The remainder other than the auxiliary component in the agent for improving low body temperature is constituted with a bulking agent consisting of a starch component like dextrin.

As the present invention examples 36 to 54 are all within the component range of the present invention described above, it was possible to confirm that the increased body temperature is high. In particular, as B group vitamins are within the aforementioned range in the present invention example 41 to 43, the increased body temperature is higher. Meanwhile, the present invention examples 40 and 44 are an example in which the added B group vitamins are not within the aforementioned range, and it showed lower increased temperature compared to the present invention examples 41 to 43 while higher increased temperature is obtained compared to the comparative examples. In addition, since Piper longum extract is contained in addition to B group vitamins in the present invention example 50, the increased body temperature is higher compared to the present invention examples 41 to 43.

The present invention examples 45 to 49 are an example in which Piper longum extract is added in addition to lactoferrin, phosphoryl oligosaccharides of calcium, and magnesium sulfate, and all were confirmed to have an increase in body temperature. Among them, as the present invention examples 46 to 48 are within the 10 to 30% range defined by the present invention, a particularly high increase in body temperature was confirmed. A high increase in body temperature was confirmed also from the present invention example 49, but there was a report from the panelist stating that the stimulation is strong.

Furthermore, the present invention example 51 is an example in which Piper longum extract is added in addition to lactoferrin and phosphoryl oligosaccharides of calcium, and the present invention example 52 is an example in which B group vitamins are added in addition to lactoferrin and phosphoryl oligosaccharides of calcium. Both were confirmed to have an increase in body temperature.

The comparative examples 17 and 18 are a so-called bulk state sample in which the auxiliary component consists only of phosphoryl oligosaccharides of calcium. Similarly, the comparative examples 19 and 20 are a sample in which the auxiliary component consists of lactoferrin. An increase in body temperature was not shown from any of the comparative examples 17 to 20. That is because, since only phosphoryl oligosaccharides of calcium is contained in the comparative examples 17 and 18, a material capable of contributing to body temperature increase was not contained at all. In addition, according to the comparative examples 19 and 20, lactoferrin, which is capable of contributing to body temperature increase, by itself cannot be decomposed by gastric juice to arrive at intestine, and thus the desired body temperature increasing effect cannot be exhibited.

According to the comparative examples 21 to 23, phosphoryl oligosaccharides of calcium and lactoferrin are all within the range defined by the present invention, but no magnesium sulfate is contained therein. Due to such reasons, the body temperature increasing effect in human body which is caused by magnesium ions obtained by decomposition of magnesium sulfate cannot be obtained, and thus it is believed that the activity of having lactoferrin in an agglomerated state so as to prevent decomposition by gastric juice cannot be exhibited.

According to the comparative example 24, phosphoryl oligosaccharides of calcium and magnesium sulfate are all within the range defined by the present invention, but no lactoferrin is contained therein.

According to the comparative example 25, phosphoryl oligosaccharides of calcium and magnesium sulfate are all within the range defined by the present invention, but the content of lactoferrin is higher than the upper limit. Due to such reasons, the body temperature increasing effect can be confirmed but the increased temperature is not high compared to the present invention examples.

According to the comparative example 26, phosphoryl oligosaccharides of calcium and magnesium sulfate are all within the range defined by the present invention, but the content of lactoferrin is lower than the lower limit. Due to such reasons, the body temperature increasing effect can be confirmed but the increased temperature is not high compared to the present invention examples.

According to the comparative example 27, lactoferrin and magnesium sulfate are all within the range defined by the present invention, but the content of phosphoryl oligosaccharides of calcium is higher than the upper limit. Due to such reasons, the body temperature increasing effect can be confirmed but the increased temperature is not high compared to the present invention examples.

According to the comparative example 28, lactoferrin and phosphoryl oligosaccharides of calcium are all within the range defined by the present invention, but the content of magnesium sulfate is lower than the lower limit. Due to such reasons, the body temperature increasing effect can be confirmed but the increased temperature is not high compared to the present invention examples.

According to the comparative example 29, lactoferrin and phosphoryl oligosaccharides of calcium are all within the range defined by the present invention, but the content of magnesium sulfate is higher than the upper limit. Due to such reasons, the body temperature increasing effect can be confirmed but there were 2 panelists complaining an upset stomach and a symptom of watery stool as caused by addition of excessive magnesium sulfate.

Furthermore, as a result of receiving the response from the panelists A and B who had been administered with the comparative example 22 and the present invention example 37, respectively, it was reported that feeling of faster body temperature increase is obtained with the present invention example 37. Based on this, an effect showing that the body temperature of a subject is more rapidly increased based on magnesium sulfate and thereafter the body temperature of a subject is more slowly increased based on lactoferrin was exhibited.

Furthermore, as a result of receiving the response from the panelists A and B who had been administered with the comparative example 22 and the present invention example 37, respectively, it was reported that there was feeling of having high body temperature for a long period of time. Based on this, a 3-step temperature increasing effect showing that the body temperature of a subject is more rapidly increased based on magnesium sulfate, the body temperature of a subject is more slowly increased based on lactoferrin thereafter, and the body temperature of a subject is increased thereafter based on B group vitamins was confirmed.

### Reference Signs List

- 1: Nucleating agent
- 2, 12: Auxiliary component
- 6: Patient
- 10: Tablet
- 11: Main agent
- 20: Chemical composition
- 12: Auxiliary component
- 16: Subject
- 31: Oral fitting body
- 32: Charging pocket

## Claims

1. An oral care composition for improving the health of an oral cavity wherein the composition is to be held in an oral cavity, the oral care composition comprising, in terms of % by mass relative to the total mass of the composition, phosphoryl oligosaccharides of calcium: 1% to less than 30% and lactoferrin: 0.01 to 10%.

2. The oral care composition according to claim 1, further comprising sodium bicarbonate: 1% to 20%.

3. The oral care composition according to claim 1 or 2, further comprising potassium carbonate or potassium hydrogen carbonate: 1% to 5%.

4. The oral care composition according to claim 1, further comprising magnesium carbonate: 1% to 5%.

5. The oral care composition according to claim 1, further comprising sucralose: 1% to 5%.

## Patentansprüche

1. Mundpflegezusammensetzung zur Verbesserung der Gesundheit einer Mundhöhle, wobei die Zusammensetzung in einer Mundhöhle zu halten ist, wobei
die Mundpflegezusammensetzung, bezogen auf Massen-% bezogen auf die Gesamtmasse der Zusammensetzung, Phosphoryloligosaccharide von Calcium umfasst: 1 % bis weniger als 30% und Lactoferrin: 0,01 bis 10%.

2. Mundpflegezusammensetzung nach Anspruch 1, ferner umfassend Natriumbicarbonat: 1% bis 20%.

3. Mundpflegezusammensetzung nach Anspruch 1 oder 2, ferner umfassend Kaliumcarbonat oder Kaliumhydrogencarbonat: 1% bis 5%.

4. Mundpflegezusammensetzung nach Anspruch 1, ferner umfassend Magnesiumcarbonat: 1% bis 5%.

5. Mundpflegezusammensetzung nach Anspruch 1, ferner umfassend Sucralose: 1% bis 5%.

## Revendications

1. Composition d'hygiène buccale pour améliorer l'état de santé d'une cavité buccale, laquelle composition est destinée à être maintenue dans la cavité buccale,
la composition d'hygiène buccale comprenant, en % en masse par rapport à la masse totale de la composition, 1 % à moins de 30 % d'oligosaccharides phosphorylés de calcium, et 0,01 à 10 % de lactoferrine.

2. Composition d'hygiène buccale selon la revendication 1, comprenant en outre 1 % à 20 % de bicarbonate de sodium.

3. Composition d'hygiène buccale selon la revendication 1 ou 2, comprenant en outre 1 % à 5 % de carbonate de potassium ou d'hydrogénocarbonate de potassium.

4. Composition d'hygiène buccale selon la revendication 1, comprenant en outre 1 % à 5 % de carbonate de magnésium.

5. Composition d'hygiène buccale selon la revendication 1, comprenant en outre 1 % à 5 % de sucralose.
